(19) Europäisches Patentamt
European Patent Office
Office européen des brevets

(11) **EP 3 420 360 B1**

(12) **FASCICULE DE BREVET EUROPEEN**

(45) Date de publication et mention de la délivrance du brevet:
**11.11.2020 Bulletin 2020/46**

(21) Numéro de dépôt: **17702906.3**

(22) Date de dépôt: **03.01.2017**

(51) Int Cl.:
**G01N 33/68** *(2006.01)* **G01N 33/74** *(2006.01)*

(86) Numéro de dépôt international:
**PCT/FR2017/050002**

(87) Numéro de publication internationale:
**WO 2017/144788 (31.08.2017 Gazette 2017/35)**

(54) **PROCEDE DE DETECTION D'ACCOUCHEMENT IMMINENT**

VERFAHREN ZUR DETEKTION VON BEVORSTEHENDER ENTBINDUNG

METHOD FOR DETECTING IMMINENT CHILDBIRTH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.02.2016 FR 1651626**

(43) Date de publication de la demande:
**02.01.2019 Bulletin 2019/01**

(73) Titulaire: **Biosynex**
**67400 Illkirch-Graffenstaden (FR)**

(72) Inventeurs:
• **PAPER, Thierry**
**67000 Strasbourg (FR)**
• **BLIN, Capucine**
**67202 Wolfisheim (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
• **Merja Kurkinen-Räty ET AL: "Combination of cervical interleukin-6 and -8, phosphorylated insulin-like growth factor-binding protein-1 and transvaginal cervical ultrasonography in assessment of the risk of preterm birth", British Journal of Obstetrics and Gynaecology, vol. 108, no. 8 1 août 2001 (2001-08-01), pages 875-881, XP055324315, Extrait de l'Internet: URL:http://onlinelibrary.wiley.com/store/1 0.1111/j.1471-0528.2001.00199.x/asset/j.14 71-0528.2001.00199.x.pdf?v=1&t=iw3p20bc&s= 09d7026a42842004c7e6c7f454979fddcc9d9668 [extrait le 2016-12-01] cité dans la demande**
• **GEERT ZEGELS ET AL: "Use of cervicovaginal fluid for the identification of biomarkers for pathologies of the female genital tract", PROTEOME SCIENCE, vol. 8, no. 1, 1 janvier 2010 (2010-01-01) , page 63, XP055065859, ISSN: 1477-5956, DOI: 10.1186/1477-5956-8-63**
• **FRANCESCA RIBONI ET AL: "Combination of biochemical markers in predicting pre-term delivery", ARCHIVES OF GYNECOLOGY AND OBSTETRICS, SPRINGER, BERLIN, DE, vol. 285, no. 1, 3 mai 2011 (2011-05-03), pages 61-66, XP019994652, ISSN: 1432-0711, DOI: 10.1007/S00404-011-1915-9**

- G Eleje ET AL: "Evaluation de Premaquick, nouveau test multiplex pronostic d'accouchement imminent chez des patientes avec menace d'accouchement prématuré", Les journées nationales - Collège national des gynécologues et obstétriciens français, 2 décembre 2016 (2016-12-02), page 82, XP055355789, Extrait de l'Internet: URL:http://www.cngof-congres.fr/sites/defa ult/files/u3/eposters.pdf [extrait le 2017-03-16]
- ELEJE GEORGE UCHENNA ET AL: "Accuracy of a combined insulin-like growth factor-binding protein-1/interleukin-6 test (Premaquick) in predicting delivery in women with threatened preterm labor", JOURNAL OF PERINATAL MEDICINE, WALTER DE GRUYTER GMBH, DE, vol. ahead of print, 25 février 2017 (2017-02-25), pages 1-10, XP008183569, ISSN: 1619-3997, DOI: 10.1515/JPM-2016-0339
- Anonymous: "igfbp1a insulin-like growth factor binding protein 1a [Danio rerio (zebrafish)] - Gene - NCBI", , 1 janvier 2002 (2002-01-01), XP055325275, Extrait de l'Internet: URL:https://www.ncbi.nlm.nih.gov/gene/3176 38 [extrait le 2016-12-01]
- Anonymous: "igfbp1b insulin-like growth factor binding protein 1b [Danio rerio (zebrafish)] - Gene - NCBI", , 1 janvier 2008 (2008-01-01), XP055325276, Extrait de l'Internet: URL:https://www.ncbi.nlm.nih.gov/gene/7939 07 [extrait le 2016-12-01]
- RUTANEN E-M ET AL: "EVALUATION OF A RAPID STRIP TEST FOR INSULIN-LIKE GROWTH FACTOR BINDING PROTEIN-1 IN THE DIAGNOSIS OF RUPTURED FETAL MEMBRANES", CLINICA CHIMICA ACTA, ELSEVIER BV, AMSTERDAM, NL, vol. 253, no. 1/02, 1 January 1996 (1996-01-01), pages 91-101, XP000874594, ISSN: 0009-8981, DOI: 10.1016/0009-8981(96)80001-E

SEQUENCE LISTING

<110> biosynex

<120> PROCEDE DE DETECTION/PREDICTION D'ACCOUCHEMENT IMMINENT

<130> BNT220689PC01

<150> FR1651626
<151> 2016-02-26

<160> 6

<170> PatentIn version 3.5

<210> 1
<211> 259
<212> PRT
<213> Homo sapiens

<400> 1

```
Met Ser Glu Val Pro Val Ala Arg Val Trp Leu Val Leu Leu Leu
1               5               10              15

Thr Val Gln Val Gly Val Thr Ala Gly Ala Pro Trp Gln Cys Ala Pro
        20              25              30

Cys Ser Ala Glu Lys Leu Ala Leu Cys Pro Pro Val Ser Ala Ser Cys
        35              40              45

Ser Glu Val Thr Arg Ser Ala Gly Cys Gly Cys Cys Pro Met Cys Ala
    50              55              60

Leu Pro Leu Gly Ala Ala Cys Gly Val Ala Thr Ala Arg Cys Ala Arg
65              70              75              80

Gly Leu Ser Cys Arg Ala Leu Pro Gly Glu Gln Gln Pro Leu His Ala
            85              90              95

Leu Thr Arg Gly Gln Gly Ala Cys Val Gln Glu Ser Asp Ala Ser Ala
            100             105             110

Pro His Ala Ala Glu Ala Gly Ser Pro Glu Ser Pro Glu Ser Thr Glu
        115             120             125

Ile Thr Glu Glu Glu Leu Leu Asp Asn Phe His Leu Met Ala Pro Ser
    130             135             140

Glu Glu Asp His Ser Ile Leu Trp Asp Ala Ile Ser Thr Tyr Asp Gly
145             150             155             160

Ser Lys Ala Leu His Val Thr Asn Ile Lys Lys Trp Lys Glu Pro Cys
```

26

165                           170                           175

Arg Ile Glu Leu Tyr Arg Val Val Glu Ser Leu Ala Lys Ala Gln Glu
                180                     185                 190

Thr Ser Gly Glu Glu Ile Ser Lys Phe Tyr Leu Pro Asn Cys Asn Lys
            195                     200             205

Asn Gly Phe Tyr His Ser Arg Gln Cys Glu Thr Ser Met Asp Gly Glu
        210                 215                 220

Ala Gly Leu Cys Trp Cys Val Tyr Pro Trp Asn Gly Lys Arg Ile Pro
225             230                 235                     240

Gly Ser Pro Glu Ile Arg Gly Asp Pro Asn Cys Gln Ile Tyr Phe Asn
                245                 250                 255

Val Gln Asn

<210> 2
<211> 229
<212> PRT
<213> Homo sapiens

<400> 2

27

```
Ala Pro Cys Ser Ala Glu Lys Leu Ala Leu Cys Pro Pro Val Ser Ala
1               5                   10              15

Ser Cys Ser Glu Val Thr Arg Ser Ala Gly Cys Gly Cys Cys Pro Met
            20                  25              30

Cys Ala Leu Pro Leu Gly Ala Ala Cys Gly Val Ala Thr Ala Arg Cys
        35                  40                  45

Ala Arg Gly Leu Ser Cys Arg Ala Leu Pro Gly Glu Gln Gln Pro Leu
    50                  55                  60

His Ala Leu Thr Arg Gly Gln Gly Ala Cys Val Gln Glu Ser Asp Ala
65                  70                  75                  80

Ser Ala Pro His Ala Ala Glu Ala Gly Ser Pro Glu Ser Pro Glu Ser
            85                  90                  95

Thr Glu Ile Thr Glu Glu Glu Leu Leu Asp Asn Phe His Leu Met Ala
            100                 105                 110

Pro Ser Glu Glu Asp His Ser Ile Leu Trp Asp Ala Ile Ser Thr Tyr
            115                 120                 125

Asp Gly Ser Lys Ala Leu His Val Thr Asn Ile Lys Lys Trp Lys Glu
    130                 135                 140

Pro Cys Arg Ile Glu Leu Tyr Arg Val Val Glu Ser Leu Ala Lys Ala
145                 150                 155                 160

Gln Glu Thr Ser Gly Glu Glu Ile Ser Lys Phe Tyr Leu Pro Asn Cys
            165                 170                 175

Asn Lys Asn Gly Phe Tyr His Ser Arg Gln Cys Glu Thr Ser Met Asp
            180                 185                 190

Gly Glu Ala Gly Leu Cys Trp Cys Val Tyr Pro Trp Asn Gly Lys Arg
            195                 200                 205

Ile Pro Gly Ser Pro Glu Ile Arg Gly Asp Pro Asn Cys Gln Ile Tyr
    210                 215                 220

Phe Asn Val Gln Asn
225
```

<210> 3
<211> 258
<212> PRT
<213> Homo sapiens

<400> 3

Ser Glu Val Pro Val Ala Arg Val Trp Leu Val Leu Leu Leu Thr
1               5                   10                  15

Val Gln Val Gly Val Thr Ala Gly Ala Pro Trp Gln Cys Ala Pro Cys
            20                  25                  30

Ser Ala Glu Lys Leu Ala Leu Cys Pro Pro Val Ser Ala Ser Cys Ser
            35                  40                  45

Glu Val Thr Arg Ser Ala Gly Cys Gly Cys Cys Pro Met Cys Ala Leu
            50                  55                  60

Pro Leu Gly Ala Ala Cys Gly Val Ala Thr Ala Arg Cys Ala Arg Gly
65                  70                  75                  80

Leu Ser Cys Arg Ala Leu Pro Gly Glu Gln Gln Pro Leu His Ala Leu
                85                  90                  95

Thr Arg Gly Gln Gly Ala Cys Val Gln Glu Ser Asp Ala Ser Ala Pro

|  | 100 | | | 105 | | | 110 | |

His Ala Ala Glu Ala Gly Ser Pro Glu Ser Pro Glu Ser Thr Glu Ile
115 120 125

Thr Glu Glu Glu Leu Leu Asp Asn Phe His Leu Met Ala Pro Ser Glu
130 135 140

Glu Asp His Ser Ile Leu Trp Asp Ala Ile Ser Thr Tyr Asp Gly Ser
145 150 155 160

Lys Ala Leu His Val Thr Asn Ile Lys Lys Trp Lys Glu Pro Cys Arg
165 170 175

Ile Glu Leu Tyr Arg Val Val Glu Ser Leu Ala Lys Ala Gln Glu Thr
180 185 190

Ser Gly Glu Glu Ile Ser Lys Phe Tyr Leu Pro Asn Cys Asn Lys Asn
195 200 205

Gly Phe Tyr His Ser Arg Gln Cys Glu Thr Ser Met Asp Gly Glu Ala
210 215 220

Gly Leu Cys Trp Cys Val Tyr Pro Trp Asn Gly Lys Arg Ile Pro Gly
225 230 235 240

Ser Pro Glu Ile Arg Gly Asp Pro Asn Cys Gln Ile Tyr Phe Asn Val
245 250 255

Gln Asn

<210> 4
<211> 137
<212> PRT
<213> Homo sapiens

<400> 4

```
Ala Pro Trp Gln Cys Ala Pro Cys Ser Ala Glu Lys Leu Ala Leu Cys
1               5               10                  15

Pro Pro Val Ser Ala Ser Cys Ser Glu Val Thr Arg Ser Ala Gly Cys
            20              25                  30

Gly Cys Cys Pro Met Cys Ala Leu Pro Leu Gly Ala Ala Cys Gly Val
        35              40                  45

Ala Thr Ala Arg Cys Ala Arg Gly Leu Ser Cys Arg Ala Leu Pro Gly

    50              55                  60

Glu Gln Gln Pro Leu His Ala Leu Thr Arg Gly Gln Gly Ala Cys Val
65              70              75                  80

Gln Glu Ser Asp Ala Ser Ala Pro His Ala Ala Glu Ala Gly Ser Pro
            85              90                  95

Glu Ser Pro Glu Ser Thr Glu Ile Thr Glu Glu Glu Leu Leu Asp Asn
            100             105                 110

Phe His Leu Met Ala Pro Ser Glu Glu Asp His Ser Ile Leu Trp Asp
            115             120                 125

Ala Ile Ser Thr Tyr Asp Gly Ser Lys
    130             135
```

<210> 5
<211> 162
<212> PRT
<213> Homo sapiens

<400> 5

```
Met Ser Glu Val Pro Val Ala Arg Val Trp Leu Val Leu Leu Leu Leu
1               5                   10                  15

Thr Val Gln Val Gly Val Thr Ala Gly Ala Pro Trp Gln Cys Ala Pro
            20                  25                  30

Cys Ser Ala Glu Lys Leu Ala Leu Cys Pro Pro Val Ser Ala Ser Cys
        35                  40                  45

Ser Glu Val Thr Arg Ser Ala Gly Cys Gly Cys Cys Pro Met Cys Ala
    50                  55                  60

Leu Pro Leu Gly Ala Ala Cys Gly Val Ala Thr Ala Arg Cys Ala Arg
65                  70                  75                  80

Gly Leu Ser Cys Arg Ala Leu Pro Gly Glu Gln Gln Pro Leu His Ala
            85                  90                  95

Leu Thr Arg Gly Gln Gly Ala Cys Val Gln Glu Ser Asp Ala Ser Ala
            100                 105                 110

Pro His Ala Ala Glu Ala Gly Ser Pro Glu Ser Pro Glu Ser Thr Glu
        115                 120                 125

Ile Thr Glu Glu Glu Leu Leu Asp Asn Phe His Leu Met Ala Pro Ser


    130                 135                 140

Glu Glu Asp His Ser Ile Leu Trp Asp Ala Ile Ser Thr Tyr Asp Gly
145                 150                 155                 160

Ser Lys
```

<210> 6
<211> 161
<212> PRT
<213> Homo sapiens

<400> 6

```
Ser Glu Val Pro Val Ala Arg Val Trp Leu Val Leu Leu Leu Leu Thr
1               5                   10                  15

Val Gln Val Gly Val Thr Ala Gly Ala Pro Trp Gln Cys Ala Pro Cys
            20                  25                  30

Ser Ala Glu Lys Leu Ala Leu Cys Pro Pro Val Ser Ala Ser Cys Ser
        35                  40                  45

Glu Val Thr Arg Ser Ala Gly Cys Gly Cys Cys Pro Met Cys Ala Leu
    50                  55                  60

Pro Leu Gly Ala Ala Cys Gly Val Ala Thr Ala Arg Cys Ala Arg Gly
65                  70                  75                  80

Leu Ser Cys Arg Ala Leu Pro Gly Glu Gln Gln Pro Leu His Ala Leu
            85                  90                  95

Thr Arg Gly Gln Gly Ala Cys Val Gln Glu Ser Asp Ala Ser Ala Pro
            100                 105                 110

His Ala Ala Glu Ala Gly Ser Pro Glu Ser Pro Glu Ser Thr Glu Ile
    115                 120                 125

Thr Glu Glu Glu Leu Leu Asp Asn Phe His Leu Met Ala Pro Ser Glu
    130                 135                 140

Glu Asp His Ser Ile Leu Trp Asp Ala Ile Ser Thr Tyr Asp Gly Ser
145                 150                 155                 160

Lys
```

## Description

### Domaine technique

La présente invention se rapporte à un procédé de détection/prédiction in-vitro d'accouchement imminent et à un dispositif de mise en œuvre du dit procédé.

La présente invention trouve, notamment une application dans le domaine médical, le domaine diagnostique, en particulier dans le domaine obstétrique.

Dans la description ci-dessous, les références entre crochets ([ ]) renvoient à la liste des références présentée à la fin du texte.

### Etat de la technique

Durant la grossesse, les femmes peuvent être soumises à différents facteurs et/ou évènements susceptibles de mettre en danger la grossesse et/ou de provoquer un accouchement prématuré ou de déclencher l'accouchement.

Un accouchement prématuré est un facteur de risque à la fois pour la mère mais également pour l'enfant à naitre.

Un accouchement prématuré est dans 50% des cas précédé d'une menace d'accouchement prématuré c'est-à-dire la survenue d'association de modifications cervicales et de contractions utérines régulières et douloureuses entre les 22 et 36ème semaines d'aménorrhées. Les accouchements prématurés sont responsable de 75% la mortalité néonatale, de la moitié des déficits neurologiques et représente la première cause d'hospitalisation en cours de grossesse, soit environ 60 000 hospitalisations par an.

En fonction de la date d'accouchement, et de l'âge du fœtus, il existe différents types de maternités afin d'assurer les soins correspondants aux nouveau-nés.

Les maternités de niveau disposent d'une unité d'obstétrique et permettent la prise en charge des grossesses normales. Ces maternités assurent une présence pédiatrique permettant l'examen du nouveau-né et la prise en charge auprès de la mère d'un certain nombre de situations fréquentes et sans gravité.

Les maternités de niveau II disposent d'une unité d'obstétrique et d'une unité de néonatalogie. Elles permettent une prise en charge des grossesses à risques modérés et des nouveau-nés nécessitant une surveillance particulière, mais pas de soins en réanimation.

Les maternités de niveau III disposent d'une unité d'obstétrique, d'une unité de néonatalogie et d'une unité de réanimation néonatale. Elles permettent une prise en charge des grossesses à haut risque et des nouveau-nés présentant un risque à la naissance, il peut s'agir notamment des grands prématurés moins de 33 semaines.

Un accouchement prématuré peut se produire chez des femmes dites à risque, par exemple présentant une hypertension, un diabète gestationnelle, mais également chez des femmes ne présentant pas de tels facteurs, dite à faible risques.

De nombreuses études et recherche ont été réalisées sur le travail et/ou l'accouchement prématuré. Toutefois, il n'existe pas actuellement de moyens/procédé totalement fiable de prédiction de ce travail/accouchement prématuré. Les moyens connus pour le traitement du travail/accouchement prématuré sont des moyens pharmacologiques qui sont utilisés une fois le travail en cours. Il s'agit par exemple dans le cas de travail prématuré à 30 semaines d'aménorrhées, de protocole pharmacologique avec administration de tocolytique, par exemple du tractocile pendant 48 heures, de nifédipine, accompagné le cas échéant d'une administration de corticoïdes pour la maturation des poumons du fœtus.

Il existe donc un réel besoin de trouver un procédé / un moyen de prédiction/détection d'accouchement imminent pour mieux contrôler le risque de prématurité, en particulier un procédé permettant une prise en charge anticipée/rapide des femmes enceintes.

Des recherches de « marqueurs » ont été effectuées afin d'essayer d'élaborer / d'identifier des marqueurs utiles pour la prédiction d'accouchement prématuré. Par exemple, la longueur du col échographique, la présence de la fibronectine fœtale, IGFBP-1 et de la PAMG-1 dans les sécrétions cervico-vaginales ont été étudiés. L'IGFBP-1 et de la fibronectine sont considérés comme des marqueurs de maturité du col. Il a été décrit qu'une combinaison de ces marqueurs pourraient éventuellement améliorer la prédiction d'un accouchement prématuré [6]. En particulier, Eroglu et al a décrit que la combinaison de la fibronectine / IGFBP-1 et de la longueur échographique du col <25 mm a permis d'augmenter la spécificité et la valeur prédictive positive pour un accouchement dans les sept jours [1]. Toutefois, l'utilité, la mise en œuvre d'un tel test n'est pas pertinente voire possible dans la mesure où elle requiert une échographie et un expert dans l'interprétation de l'imagerie échographique qui représente clairement un obstacle à son utilisation [2,3]. Une autre étude réalisée par Goepfert décrit qu'un test utilisant IL-6 et la fibronectine a montré une corrélation [4]. Toutefois une méta-analyse de l'utilité de la fibronectine dans la prédiction de la prématurité dans les 7-14 jours chez les femmes symptomatiques a mis en avant une sensibilité de 78-89% et une spécificité de 86% mais une sensibilité plus faible (68-76%) et une spécificité comparables (88-89%) dans les femmes asymptomatiques [5]. En outre les test utilisant la fibronectine ont mis en exergue une très faible valeur prédictive positive [6] et des limites d'utilisations/résultats faussés en raison de facteurs externes tel que par exemple des saignements vaginaux et/ou des rapports sexuels récents non protégés [7].

Des procédés utilisant différents marqueurs ont été envisagés afin de prédire un accouchement prématuré. Par exemple Kurkinen-Räty et al. ont décrit que l'augmentation de col de l'utérus, de la concentration d'IL-6 et de la longueur du col par mesure échographique semble être associée à la prématurité [8]. Cependant, ni la sensibilité ni la spécificité des tests utilisés dans l'étude Kurkinen-Räty n'était suffisante pour prédire l'accouchement prématuré pour la bonne prise de décision clinique. Riboni et al. ont décrit un procédé de détection/prédiction in vitro d'un accouchement imminent comprenant une étape de recherche, dans un prélèvement de sécrétions vaginales ou cervicales, de l'IGFBP-1 et de l'interleukine 6 [14].

De plus des méthodes telles que l'élastographie du col ou la mesure des mouvements respiratoire fœtaux ont fait également l'objet d'étude comme potentiel marqueurs d'accouchement imminent ou prématuré. Concernant l'élastographie du col, des études ont montré une modification de la cartographie avant et après maturation du col=, mais également entre 16 et 24 SA un lien entre les mesures effectuées et un risque d'accouchement prématuré. Toutefois, ce critère est clairement examinateur dépendant concernant les mesures effectuées et il n'existe pas actuellement de valeur standard permettant de conclure.

Les mouvements respiratoires fœtaux (MRF) ont été également étudiés comme un possible marqueur du risque d'accouchement imminent / prématuré. En effet, il est connu que les MRF diminuent ou cessent dans les 24 à 36 heures qui précèdent l'entrée en travail. Des études ont donc été réalisées pour mesurer la prédictivité de l'accouchement à 48 heures sur la base des MRF. Ce procédé a montré des résultats prédictifs avec une sensibilité et spécificité similaires aux procédés usuels qui ne permettent pas de s'affranchir d'un examen clinique et/ou d'éviter l'administration de traitement non requis.

Il existe donc un réel besoin de trouver un procédé / un moyen de prédiction/détection d'accouchement imminent, ne présentant pas les inconvénients/limites des procédés connus. En particulier un procédé/moyen permettant d'obtenir des résultats avec une sensibilité/spécificité/valeur prédictive positive et négative compatibles avec une prise en charge améliorée du risque de prématurité.

Il existe également un réel besoin de trouver un procédé / moyen de prédiction/détection d'accouchement prématuré dont l'utilisation/les résultats ne varient pas en fonction de facteurs externes, par exemple de saignements vaginaux éventuels et/ou de la proximité de rapports sexuels.

Par ailleurs, il est connu qu'il existe une grande hétérogénéité dans les signes / bilans cliniques et dans les mécanismes biochimiques impliqués dans l'accouchement prématuré complexifiant ainsi sa détection/préd iction.

Il existe donc également un réel besoin de trouver un procédé / moyen de prédiction/détection d'accouchement prématuré dont l'utilisation/les résultats sont fiables quel que soit les signes cliniques et /ou mécanismes biochimiques impliquées.

## Description de l'invention

La présente invention a précisément pour but de répondre à ces besoins et inconvénients de l'art antérieur en fournissant un procédé de détection/prédiction in-vitro d'accouchement imminent.

En particulier, la présente invention a pour objet un procédé de détection/prédiction in-vitro d'un accouchement imminent, comprenant une étape de recherche simultanée, dans un prélèvement de sécrétions vaginales ou cervicales, des marqueurs du groupe constitué de la protéine 1 de liaison au facteur de croissance insulinomimétique intacte (IGFBP-1 intacte), de la protéine 1 de liaison au facteur de croissance insulinomimétique totale comprenant la protéine 1 de liaison au facteur de croissance insulinomimétique intacte et un fragment N-Terminal de la protéine 1 de liaison au facteur de croissance insulinomimétique (IGFBP-1 totale) et de l'interleukine 6 (IL-6), un accouchement imminent est détecté/prédit lorsque au moins deux des marqueurs du groupe constitué de l'IGFBP-1 intacte, de l'IGFBP-1 totale et de l'IL-6, sont détectés.

La protéine 1 intacte de liaison au facteur de croissance Insulinomimétique (IGFBP-1A) est parfois décrite par certains auteurs sous le nom de protéine placentaire 12 (PP-12) ou Placental Alpha Microglobulin-1 (PAMG-1).

Dans la présente demande, la protéine de liaison au facteur de croissance insulinomimétique intacte ou native peut être désignée par IGFBP-1 intacte, IGFBP-1 N ou IGFBP-1 A. La protéine de liaison au facteur de croissance insulinomimétique totale, comprenant la protéine 1 de liaison au facteur de croissance insulinomimétique intacte et un fragment N-Terminal de la protéine 1 de liaison au facteur de croissance insulinomimétique peut être désignée par IGFBP-1 totale, IGFBP-1 T ou IGFBP-1 B.

Avantageusement, les inventeurs ont démontré que le procédé selon l'invention permet d'exclure un accouchement imminent lorsqu'aucun ou un seul des marqueurs sont détectés. En d'autres termes, le procédé selon l'invention permet de conclure avec fiabilité accrue sur l'absence de menace d'accouchement imminent permettant ainsi d'éviter des hospitalisations non nécessaires.

La présente invention se rapporte également à un procédé de détection/prédiction in-vitro d'un accouchement imminent dans un échantillon de sécrétions vaginales ou cervicales comprenant :

a) Recherche de la présence d'IGFBP-1 intacte, d'IGFBP-1 totale et d'IL-6,
b) Affectation d'une valeur d'IGFBP-1 intacte ($I_A$) si l'IGFBP-1 intacte est présente dans ledit échantillon ou de 0 si l'IGFBP-1 intacte n'est pas présente,
c) Affectation d'une valeur d'IGFBP1 totale ($I_B$) si l'IGFBP-1 totale est présente dans ledit échantillon ou de 0 si l'IGFBP-1 totale n'est pas présente, la valeur d'$I_B$ affectée si l'IGFBP-1 totale est présente dans ledit échantillon étant inférieure ou égale à la valeur d'$I_A$ affectée lorsque l'IGFBP-1 intacte est présente dans ledit échantillon,
d) Affectation d'une valeur d'IL-6 ($I_6$) si l'IL-6 est présente dans ledit échantillon ou de 0 si l'IL-6 n'est pas présente, la valeur $I_6$ affectée lorsqu'IL-6 est présente dans ledit échantillon étant strictement inférieure aux valeurs d'$I_A$ et $I_B$ affectées lorsque l'IGFBP-1 intacte et l'IGFBP-1 totale sont respectivement présentes dans ledit échantillon,
e) Calcul du score S1 selon la formule suivante : S1 = $I_A$ + $I_B$ + $I_6$

une valeur de S1 supérieure ou égale à la valeur d'$I_A$ ou $I_B$ respectivement affectée lorsque l'IGFBP-1 intacte ou l'IGFBP-1 totale est présente dans ledit échantillon, indiquant un accouchement imminent.

Une valeur de S1 inférieure ou égale à la valeur d'$I_6$ affectée lorsque l'IL6-est présente dans ledit échantillon, indique une absence accouchement imminent.

La valeur d'IGFBP-1 A (IA) affectée lorsqu'il est présent dans ledit échantillon est un entier naturel supérieur à 0, par exemple égale à 1, 2, 3, 4.

La valeur d'IGFBP-1 A (IA) affectée est supérieure ou égale à $I_B$. Par exemple la valeur de $I_A$ peut être égale à $I_B$, par exemple égale à 2, la valeur de $I_A$ peut être supérieure à $I_B$, par exemple égale à 3 et la valeur de $I_A$ égale à 2

La valeur d'IGFBP-1 B ($I_B$) affectée lorsqu'il est présent dans ledit échantillon est un entier naturel supérieur à 0, par exemple 1, 2, 3, 4.

La valeur d'IGFBP-1 B ($I_B$) affectée est inférieur ou égal à $I_A$. Par exemple la valeur d'$I_B$ peut être égale à $I_A$, par exemple égale à 2.

La valeur d'IL-6 ($I_6$) affectée lorsqu'elle est présente dans ledit échantillon est un entier naturel dont la valeur est strictement inférieure aux valeurs $I_A$ et $I_B$ affectées.

Un procédé de détection/prédiction in-vitro d'un accouchement imminent dans un échantillon de sécrétions vaginales ou cervicales est décrit. Il comprend

a) Recherche de la présence d'IGFBP-1 A, d'IGFBP-1 B et d'IL-6
b) Affectation d'une valeur d'IGFBP-1 A (IA) égale à 2 si l'IGFBP-1 A est présent dans ledit échantillon ou de 0 si l'IGFBP-1 A n'est pas présent,
c) Affectation d'une valeur d'IGFBP1 B ($I_B$) égale à 2 si l'IGFBP-1 B est présent dans ledit échantillon ou de 0 si l'IGFBP-1 B n'est pas présent,
d) Affectation d'une valeur d'IL-6 ($I_6$) égale à 1 si l'IL-6 est présente dans ledit échantillon ou de 0 si l'IL-6 n'est pas présente
e) Calcul du score S1 selon la formule suivante :

$$S_1 = I_A + I_B + I_6$$

une valeur de S1 supérieure ou égale à 2 indiquant un accouchement imminent.

Un procédé de détection/prédiction in-vitro d'un accouchement imminent dans un échantillon de sécrétions vaginales ou cervicales est décrit. Il comprend

a) Recherche de la présence d'IGFBP-1 A, d'IGFBP-1 B et d'IL-6
b) Affectation d'une valeur d'IGFBP-1 A (IA) égale à 2 si l'IGFBP-1 A est présent dans ledit échantillon ou de 0 si l'IGFBP-1 A n'est pas présent,
c) Affectation d'une valeur d'IGFBP1 B ($I_B$) égale à 2 si l'IGFBP-1 B est présent dans ledit échantillon ou de 0 si l'IGFBP-1 B n'est pas présent,
d) Affectation d'une valeur d'IL-6 ($I_6$) égale à 1 si l'IL-6 est présente dans ledit échantillon ou de 0 si l'IL-6 n'est pas présente
e) Calcul du score S1 selon la formule suivante :

$$S_1 = I_A + I_B + I_6$$

une valeur de S1 inférieure ou égale à 1 indiquant une absence accouchement imminent.

Par accouchement imminent on entend un accouchement dans un délai de 24 heures à 15 jours, par exemple dans un délai de 14 jours, dans un délai de 7 jours, dans un délai de 48 heures.

Dans la présente un prélèvement vaginal ou cervical est également désigné par échantillon de sécrétions vaginales ou cervicales.

Par prélèvement vaginal ou cervical on entend, par exemple un prélèvement effectué au niveau de la paroi du vagin d'une femme enceinte, au niveau du fornix postérieur du vagin, au niveau du col des sécrétions vaginales ou cervico-vaginales.

Le prélèvement peut être obtenu par tout procédé connu de l'homme du métier. Le prélèvement peut être obtenu, par exemple par pipetage à l'aide d'une pipette ou par frottement des parois de la cavité vaginale, par exemple avec un écouvillon stérile dont le bouton peut être du coton ou une fibre synthétique. Il peut s'agir par exemple de polyester.

L'échantillon peut être un échantillon prélevé entre la 18ème et la 42ème semaine d'aménorrhée, par entre la 22ème et la 36ème semaine.

En d'autres termes, le prélèvement peut être un échantillon prélevé entre la 18ème et la 42ème semaine d'aménorrhée, par exemple entre la 22ème et la 36ème semaine.

Par IGFBP-1 intacte, ou IGFBP-1 native ou IGFBP-1 A on entend la protéine 1 de liaison au facteur de croissance Insulinomimétique (IGFBP-1) de séquence SEQ ID N°1 suivante:

MSEVPVARVWLVLLLLTVQVGVTAGAPWQCAPCSAEKLALCPPVSASCS
EVTRSAGCGCCPMCALPLGAACGVATARCARGLSCRALPGEQQPLHAL
TRGQGACVQESDASAPHAAEAGSPESPESTEITEEELLDNFHLMAPSEE
DHSILWDAISTYDGSKALHVTNIKKWKEPCRIELYRVVESLAKAQETSGEE
ISKFYLPNCNKNGFYHSRQCETSMDGEAGLCWCVYPWNGKRIPGSPEIR
GDPNCQIYFNVQN (SEQ ID N°1),

incluant les formes phosphorylées de la séquence SEQ ID NO 1

Par IGFBP-1 intacte, ou IGFBP-1 native ou IGFBP-1 A on entend également la protéine 1 de liaison au facteur de croissance Insulinomimétique (IGFBP-1) mature de séquence SEQ ID N°2 ou ses précurseurs de séquence ID N°1 ou N°3 suivantes :

APCSAEKLALCPPVSASCSEVTRSAGCGCCPMCALPLGAACGVATARC
ARGLSCRALPGEQQPLHALTRGQGACVQESDASAPHAAEAGSPESPES
TEITEEELLDNFHLMAPSEEDHSILWDAISTYDGSKALHVTNIKKWKEPCR
IELYRVVESLAKAQETSGEEISKFYLPNCNKNGFYHSRQCETSMDGEAGL
CWCVYPWNGKRIPGSPEIRGDPNCQIYFNVQN (SEQ ID N°2),

MSEVPVARVWLVLLLLTVQVGVTAGAPWQCAPCSAEKLALCPPVSASCS
EVTRSAGCGCCPMCALPLGAACGVATARCARGLSCRALPGEQQPLHAL
TRGQGACVQESDASAPHAAEAGSPESPESTEITEEELLDNFHLMAPSEE
DHSILWDAISTYDGSKALHVTNIKKWKEPCRIELYRVVESLAKAQETSGEE
ISKFYLPNCNKNGFYHSRQCETSMDGEAGLCWCVYPWNGKRIPGSPEIR
GDPNCQIYFNVQN (SEQ ID N°1),

SEVPVARVWLVLLLLTVQVGVTAGAPWQCAPCSAEKLALCPPVSASCSE
VTRSAGCGCCPMCALPLGAACGVATARCARGLSCRALPGEQQPLHALT
RGQGACVQESDASAPHAAEAGSPESPESTEITEEELLDNFHLMAPSEED

HSILWDAISTYDGSKALHVTNIKKWKEPCRIELYRVVESLAKAQETSGEEI
SKFYLPNCNKNGFYHSRQCETSMDGEAGLCWCVYPWNGKRIPGSPEIR
GDPNCQIYFNVQN (SEQ ID N°3),

incluant les isoformes et formes phosphorylées des séquences SEQ ID N°1, 2 et/ou 3.

Par IGFBP-1 B on entend l'IGFBP-1 intacte comprenant tout ou partie d'un fragment N-Terminal de la protéine 1 de liaison au facteur de croissance Insulinomimétique (IGFBP-1) mature de séquence SEQ ID N°4 ou ses précurseurs de séquence ID N°5 ou N°6 suivantes :

APWQCAPCSAEKLALCPPVSASCSEVTRSAGCGCCPMCALPLGAACGV
ATARCARGLSCRALPGEQQPLHALTRGQGACVQESDASAPHAAEAGSP
ESPESTEITEEELLDNFHLMAPSEEDHSILWDAISTYDGSK (SEQ ID N°4),
MSEVPVARVWLVLLLLTVQVGVTAGAPWQCAPCSAEKLALCPPVSASCS
EVTRSAGCGCCPMCALPLGAACGVATARCARGLSCRALPGEQQPLHAL
TRGQGACVQESDASAPHAAEAGSPESPESTEITEEELLDNFHLMAPSEE
DHSILWDAISTYDGSK (SEQ ID N°5),

SEVPVARVWLVLLLLTVQVGVTAGAPWQCAPCSAEKLALCPPVSASCSE
VTRSAGCGCCPMCALPLGAACGVATARCARGLSCRALPGEQQPLHALT
RGQGACVQESDASAPHAAEAGSPESPESTEITEEELLDNFHLMAPSEED
HSILWDAISTYDGSK (SEQ ID N°6),

incluant les isoformes et formes phosphorylées des séquences SEQ ID N° 4, 5 et/ou 6.

En d'autres termes, la protéine de liaison au facteur de croissance insulino mimétique (IGFBP-1) intacte ou native peut être indépendamment désignée IGFBP-1 N ou IGFBP-1 A.

En d'autres termes, l'IGFBP-1 intacte comprenant un fragment N-Terminal de la protéine 1 de liaison au facteur de croissance Insulinomimétique (IGFBP-1) peut être indépendamment désigné IGFBP-1 T ou IGFBP-1 B ou IGFBP-1 totale.

Les anticorps dirigés contre l'IGFBP-1 intacte (anticorps anti IGFBP-1 A) peuvent être choisis par exemple dans le groupe comprenant les anticorps décrits dans les documents Rutanen et al, Biochem Biophys Res Commun 1988 ; 152 : 208 [9] et dans Pekonen et al, J immunoassay 1989; 10 : 325-337 [10], les anticorps anti IGFBP-1 commercialisés par la société Hytest (numéro de catalogue 4IG8 ; clone G5F8 et clone C7B9).

Avantageusement, le seuil de détection de l'IGFBP-1A dans l'échantillon peut être choisi, par exemple l'IGFBP-1 A peut être considérée comme présent dans l'échantillon lorsque sa concentration est supérieure ou égale à 40 ng/ml d'IGFBP-1.

Selon l'invention, les anticorps dirigés contre l'IGFBP-1 totale (anticorps anti IGFBP-1 B) peuvent être le mélange des anticorps commercialisés par la société Hytest numéro de catalogue 4I52 ; clone G2 ; numéro de catalogue 4IG8 ; clone G5F8 et clone C7B9.

Les inventeurs ont constaté que les mélanges des anticorps ci-dessus permettent de distinguer l'IGFBP-1 , intacte de l'IGFBP-1 totale dans la mesure où les sites de liaisons des anticorps sont situés à des localisations différentes de la protéine. Par exemple, l'anticorps numéro de catalogue 4I52 (Hytest) ; clone G2 se lie au niveau N-terminale de l'IGFBP-1.

Le pH de solution comprenant les anticorps anti IGFPB-1 B peut être de 7,4.

Avantageusement, le seuil de détection de l'IGFBP-1B dans l'échantillon peut être choisi, par exemple l'IGFBP-1 B peut être considérée comme présent dans l'échantillon lorsque sa concentration est supérieure ou égale à 20 ng/ml d'IGFBP-1 intacte et/ou de 10 ng/ml du fragment N-Ter d'IGFBP-1.

Les anticorps dirigés contre l'interleukine 6 (anticorps anti IL6) peuvent être choisis par exemple dans le groupe comprenant les anticorps commercialisé par Diaclone; numéro de catalogue 855.050.005, clone B-E8 ou B-E4.

Le pH de solution comprenant les anticorps anti IL6 peut être 7,4.

Avantageusement, le seuil de détection de l'IL-6 dans l'échantillon peut être choisi, par exemple l'IL-6 peut être considérée comme présent dans l'échantillon lorsque sa concentration est supérieure ou égale à 0,25 ng/ml.

Avantageusement, selon l'invention, la concentration de chaque anticorps et/ou le pH de chaque solution comprenant chaque anticorps peut permettre de moduler le seuil de détection des protéines IGFBP-1 A, IGFBP-1 B ou de l'interleukine 6.

Les anticorps dirigés contre l'IGFBP-1 A, IGFBP-1 B ou l'interleukine 6 peuvent être indépendamment avantageusement sous forme sèche.

Les anticorps dirigés contre l'IGFBP-1A, l'IGFBP-1 B et l'IL-6 peuvent être marqués ou non. Le marquage est exposé ci-dessous. Avantageusement, les anticorps anti-IGFBP-1 A utilisés en capture et les anticorps anti IGFBP-1 A marqués peuvent être dirigés contre des épitopes différents, les anticorps anti-IGFBP-1 B utilisés en capture et les anticorps anti IGFBP-1 B marqués peuvent être dirigés contre des épitopes différents, et les anticorps anti-IL6 utilisés en capture et les anticorps anti-IL6 marqués peuvent être dirigés contre des épitopes différents

Les anticorps dirigés contre l'IGFBP-1 A, IGFBP-1 B ou l'interleukine 6 peuvent être indépendamment marqués par tout moyen connu de l'homme du métier. Il peut s'agir par exemple d'une molécule chimique, par exemple un marqueur fluorescent, par exemple la rhodamine ou l'isothiocyanate de fluorescéine (FITC), une enzyme par exemple la peroxydase du raifort, la phosphatase alcaline, la β-galactosidase, la glucose 6-phosphate déshydrogénase, qui est révélée en présence d'un substrat spécifique par exemple le TMB (ou 3,3',5,5'-tetramethylbenzidine), le 4-Méthyl-ombelliferyl phosphate, des nanoparticules colorées, par exemple des particules de latex ou de polystyrène, des particules d'or colloïdal, des liposomes, des particules de carbone ou de sélénium, des nanoparticules fluorescentes contenant par exemple des chélates de lanthanides (Europium par exemple), ou des nanoparticules magnétiques, des molécules radioactives, par exemple de l'iode 125, l'iode 133, le tritium.

Le marquage peut être différent en fonction de la spécificité de l'anticorps. Par exemple l'anticorps anti IGFBP-1 A peut être marqué avec un marqueur, différent de celui de l'anticorps anti IGFBP-1 B, et également différent de celui de l'anticorps anti IL6. Avantageusement, la différence de marquage permet de détecter si seul l'IGFBP-1 A, seule l'IGFBP-1 B ou si seule l'interleukine 6 et/ou toute combinaison de protéines sont présentes dans l'échantillon grâce à la différence de marquage. Les marquages différents permettent de détecter de manière distincte l'IGFBP-1 A, l'IGFBP-1 B et l'interleukine 6.

Les anticorps peuvent être préalablement fixés sur un support solide. Par exemple, les anticorps dirigés contre l'IGFBP-1 A, l'IGFBP-1 B ou l'interleukine 6 peuvent être fixés sur une membrane, par exemple une membrane de nitrocellulose, le fond d'une plaque multi-puits, un papier absorbant, un support en fibre de verre et/ou toute surface connue de l'homme du métier permettant la fixation d'au moins un anticorps.

Le contrôle de la quantité d'anticorps fixée permet avantageusement d'adapter le seuil de détection de l'IGFBP-1 A, l'IGFBP-1 B et l'interleukine 6. En effet plus la quantité fixée est importante, plus le seuil de détection est faible.

En d'autres termes, anticorps peuvent être préalablement fixés sur un support, par exemple, par dépôt ou par couplage chimique.

Lorsque les anticorps sont fixés par dépôt, ils peuvent être déposés par exemple, par aspersion en une surface commune ou distincte d'anticorps anti IGFBP-1 A, anti IGFBP-1 B et anti interleukine 6 tels que définis précédemment, puis séchage par exemple, via le procédé décrit dans Beer et al. Qualification of cellulose nitrate membranes for lateral-flow assays, IVD technology, janvier 2002. [11]

Lorsque les anticorps sont fixés par couplage chimique, ils peuvent être fixés, par exemple, via le procédé décrit dans Beer et al. Qualification of cellulose nitrate membranes for lateral-flow assays, IVD technology, janvier 2002. [11]

Selon l'invention, le procédé de l'invention peut comprendre en outre une étape préliminaire de dilution du prélèvement vaginal ou cervical dans une solution de dilution. La solution de dilution peut être toute solution connue de l'homme du métier pour diluer ces prélèvements. Il peut s'agir, par exemple, d'une solution physiologique, d'une solution tampon. Il peut s'agir par exemple aussi d'une solution comprenant 50mM $Na_2HPO_4$ (S7907, SIGMA ALDRICH 1%BSA (1035-70, ID Bio) 0,05% triton X-100 0,55% Tween20 (P7949, Sigma Aldrich) (pH 7.4).

Selon l'invention, le pH de la solution de dilution peut être 5 à 10, de préférence de à 8.

La force ionique de la solution de dilution peut être de 10 à 1000 mM, de préférence de 50 à 200 mM.

Lorsque e prélèvement de sécrétions vaginales ou cervicales recueilli grâce à un écouvillon dont le bouton, il peut être préalablement dilué dans un volume de solution tampon compris de 0,5 à 2mL, par exemple 1 ml.

Le procédé peut comprendre une étape de révélation.

L'étape de révélation peut être effectuée par tout procédé connu de l'homme du métier. Il peut s'agir par exemple d'un procédé radioimmunologique (RIA), immunoradiométrique (IRMA), enzymmunologique (EIA), immunofluorométrique (IFMA), immunofluorimétrique en temps résolu, luminoimmunologique (LIA), immunomagnétique, ou par immunochromatographie ou immunofiltration grâce à des particules colorées (latex, polystyrène, or colloïdal). Certains de ces procédés peuvent être mis en œuvre comme décrit dans Anne Harwood Peruski et al. Immunological Methods for Détection and Identification of Infectious Disease and Biological Warfare Agents. Clinical and diagnostic laboratory immunology, July 2003, p. 506-513. [12]

La révélation peut être par exemple directe ou indirecte.

Lorsque la révélation est indirecte ou par compétition, on peut utiliser par exemple l'antigène IGFBP-1 A, IGFBP-1 B ou l'IL-6 marqué, par exemple à des particules, tel que décrit précédemment.

Lorsque la révélation est directe (système sandwich), on peut utiliser par exemple un anticorps de capture et un second anticorps marqué dirigé contre l'IGFBP-1 A, un anticorps de capture et un second anticorps marqué dirigé contre l'IGFBP-1 B et un anticorps de capture et un second anticorps marqué dirigé contre l'IL6 tel que décrit précédemment. Avantageusement, l'anticorps de capture et l'anticorps marqué sont dirigés contre des épitopes différents de façon à former une paire utilisable en système sandwich.

De préférence, la révélation est directe.

Avantageusement, l'intensité du signal du marqueur est fonction de la quantité et/ou de la concentration d'IGFBP-1 A, d'IGFBP-1 B et/ou d'IL6 présente dans le prélèvement.

Suivant le marqueur utilisé, la révélation peut être visuelle ou effectuée à l'aide d'un dispositif.

Lorsque le marqueur est un marqueur coloré visible tel que décrit précédemment la révélation est, avantageusement visuelle. Lorsque le marqueur est, par exemple un marqueur radioactif tel que décrit précédemment, la révélation sera effectuée, par exemple à l'aide d'un dispositif de mesure de radioactivité, par exemple un compteur geiger.

Selon l'invention, le procédé de l'invention peut comprendre un ajustement d'au moins un des paramètres choisis parmi le volume du milieu tampon diluant, le pH du milieu tampon, la force ionique du milieu tampon, ledit ajustement étant commun l'IGFBP-1A, l'IGFBP-1 B et l'IL6.

Selon l'invention, le procédé de l'invention peut comprendre un ajustement d'au moins un des paramètres choisis parmi la concentration des anticorps de capture et/ou la concentration des anticorps marqués, le dit ajustement étant spécifique à l'IGFBP-1A, l'IGFBP-1 B ou l'IL-6.

Selon l'invention, le procédé de l'invention permet avantageusement, de par la complémentarité des marqueurs de détecter/prédire un accouchement imminent chez une femme enceinte. En particulier, le procédé permet avantageusement en fonction de la présence ou l'absence des marqueurs dans l'échantillon d'identifier un sujet pour lequel un risque d'accouchement dans les 14 jours, avantageusement dans les 7 jours.

Avantageusement, les inventeurs ont démontré que le procédé selon l'invention permet la détection prédiction d'un accouchement, en particulier dans les 7 jours avec une spécificité supérieure à 93% et une sensibilité supérieure à 87%.

La présente invention permet, avantageusement, de diminuer la probabilité d'obtenir des résultats faux négatifs ainsi que des résultats faux positifs et plus particulièrement en présence de résultats triplement négatifs ou triplement positifs sur les trois paramètres inclus dans le procédé. Ainsi, le résultat obtenu permet de conclure à l'imminence de l'accouchement sans aucun test clinique complémentaire.

En outre, la présente invention permet avantageusement de détecter un accouchement prématuré quel que soit le stade de la grossesse.

En outre, avantageusement les inventeurs ont montré que lorsque les trois marqueurs, à savoir la protéine 1 intacte de liaison au facteur de croissance Insulinomimétique (IGFBP-1A), de la protéine 1 de liaison au facteur de croissance Insulinomimétique totale (incluant la forme intacte et un fragment N-Terminal) (IGFBP-1B) et de l'interleukine 6 (IL-6), sont simultanément détectés ou absents de l'échantillon/prélèvement de sécrétions vaginales ou cervicales, la prédictivité positive et négative sont respectivement accrue. Les inventeurs ont avantageusement montré que lorsque aucun des marqueurs n'est détecté, la valeur prédictive négative d'accouchement d'ici à 2 jours est de 100%, d'ici à 7 jours ou 14 jours est de 98%. Les inventeurs ont également avantageusement démontré que lorsque tous les marqueurs sont détectés, la valeur prédictive positive d'accouchement d'ici à 7 jours ou 14 jours est supérieure à 95,8%.

Avantageusement les inventeurs ont montré que lorsque le score S1 est supérieur ou égal à $I_A$, $I_A$ étant égale à la valeur affectée lorsque l'IGFBP-1 A est présent dans ledit échantillon, par exemple supérieur ou égale à 2, la valeur prédictive négative est supérieure à 96,8%. En particulier les inventeurs ont montré quel que soit l'imminence de l'accouchement, par exemple inférieur à 2 jours, inférieur à 7 jours et/ou inférieur à 14 jours, lorsque le score S1 est supérieur ou égal à $I_A$, $I_A$ étant égale à la valeurs affectée lorsque l'IGFBP-1 A est présent dans ledit échantillon, par exemple supérieur ou égale à 2, la valeur prédictive négative supérieure est supérieure à 96,8% allant jusqu'à 98,4%.

Avantageusement les inventeurs ont montré que lorsque le score S1 est égale inférieur ou égale à la valeur d'$I_6$ affecté lorsque l'IL-6 est présente dans ledit échantillon, par exemple égale à 0 ou 1, la probabilité d'accoucher d'ici à 14 jours est inférieur à 8,3% à nulle.

Avantageusement les inventeurs ont également montré que lorsque le score S1 est compris entre $I_A$ et ($I_A$ + $I_B$), les valeurs de $I_A$ et $I_B$ étant égale aux valeurs affectées lorsque l'IGFBP-1 A et l'IGFBP-1 B est présent dans ledit échantillon, par exemple compris de 2 à 4, la probabilité d'accoucher d'ici à 14 jours est supérieur à 90%.

Avantageusement les inventeurs ont également montré que lorsque le score S1 est égal à la somme des valeurs affectées lorsque l'IL6, l'IGFBP-1 A et l'IGFBP-1 B 5 sont présents dans ledit échantillon, par exemple égale à 5, la probabilité d'accoucher d'ici à 7 jours est supérieur à 95%.

Avantageusement, les inventeur ont également montré qu'un score S1 égale à 0, c'est-à-dire pour lequel aucun des marqueurs n'est détecté à une valeur prédictive négative d'accouchement d'ici à 2 jours de 100%, d'ici à 7 jours ou 14 jours de 98%. En d'autres termes, les inventeurs ont clairement démontré que le procédé de l'invention, en particulier en fonction du score S1 obtenu permet de réduire voir d'exclure totalement les résultats faux négatifs permettant avantageusement d'exclure avec certitude un accouchement imminent.

Avantageusement, les inventeurs ont également montré qu'un score S1 égal à la somme des valeurs affectées lorsque l'IL6, l'IGFBP-1 A et l'IGFBP-1 B 5 sont présents dans ledit échantillon, par exemple égale à 5, c'est-à-dire pour lequel tous les marqueurs sont détectés à une valeur prédictive positive d'accouchement d'ici à 7 jours ou 14 jours supérieur à 95,8%. En d'autres termes, les inventeurs ont clairement démontré que le procédé de l'invention, en particulier en fonction du score S1 obtenu permet de conclure à l'imminence de l'accouchement avec une prédictivité très élevée.

Le procédé peut également comprendre une étape de mesure de la longueur du col de l'utérus sur une image obtenue par échographie.

Lorsque la longueur du col de l'utérus est mesurée sur une image obtenue par échographie, une longueur du col de l'utérus compris entre 20 et 30 mm, de préférence inférieure à 25 mm est considérée comme augmentant le risque d'accouchement imminent

Le procédé peut également comprendre une étape de mesure la dilatation cervicale sur une image obtenue par échographie.

Lorsque la dilatation cervicale mesurée sur une image obtenue par échographie est supérieure ou égale à 3 cm est considérée comme augmentant le risque d'accouchement imminent.

L'image échographique peut être obtenue par tout dispositif connu de l'homme du métier. Il peut s'agir par exemple d'un dispositif disponible dans le commerce, par exemple commercialisé par la société GE Healthcare, Philips.

L'image échographique peut être obtenu préalablement ou postérieurement à l'étape de recherche de l'IGFBP-1 A, l'IGFBP-1 B et/ou d'IL-6. Par exemple l'image échographique peut être obtenue 1 à 12 heures préalablement à l'étape de recherche ou de 0,5 à 12 heures postérieurement à l'étape de recherche

De préférence, la mesure de la dilatation du col est réalisée postérieurement à l'étape de recherche de l'IGFBP-1 A, l'IGFBP-1 B et/ou d'IL-6.

Avantageusement, le procédé peut être également utilisé pour détecter/mesurer un risque de rupture prématurée des membranes fœtales.

En outre, le procédé permet également avantageusement de détecter/mesurer un risque de rupture prématurée des membranes fœtales.En particulier, un accouchement prématuré impliquant une rupture de la membrane fœtale, la prédiction/détection d'un accouchement imminent permet également de détecter un risque de rupture prématurée des membranes fœtales.

En particulier, dans ce mode de réalisation, lorsque l'IGFBP-1A et/ou l'IGFBP-1 B et/ou l'IL-6 sont détectés dans l'échantillon, cela peut impliquer notamment un risque de rupture de membranes fœtales.

Avantageusement, le procédé peut être également utilisé pour déterminer la période optimale de déclenchement de l'accouchement.

La présente invention a également pour objet un dispositif, par exemple immunochromatographique, pour la mise en œuvre du procédé selon l'invention comprenant :

- une zone (1) de dépôt d'un échantillon
- une zone (2) comprenant des anticorps anti IGFBP-1 intacte et des anticorps anti IGFBP-1 totale et des anticorps anti IL-6 marqués.
- une zone (3) de révélation comprenant des anticorps de capture dirigés contre IGFBP-1 intacte
- une zone (4) de révélation comprenant des anticorps de capture dirigés contre l'IGFPB-1 totale
- une zone (5) de révélation comprenant des anticorps de capture dirigés contre IL-6.
- Eventuellement, une zone (6) d'absorption du fluide

La zone (1) de dépôt peut être une zone adaptée à l'application ou la réception du prélèvement. Cette zone peut être de toute forme connue de l'homme du métier, par exemple un réservoir, une cupule, un puits, une mèche, une surface plane.

La zone (1) de dépôt peut être mobile et/ou liée à l'une quelconque des zones (2) à (5). Lorsque la zone (1) est mobile, elle peut être, par exemple, utilisée pour effectuer le prélèvement et appliquée sur l'une ou l'ensemble des zones (2) à (5). Lorsque la zone (1) est liée à l'une des zones (2) à (5) celle-ci peut être plongée directement dans un récipient comprenant le prélèvement et/ou le prélèvement peut être appliqué sur cette zone.

Les matériaux des zones (1) à (5) peuvent être identiques ou différents. Les matériaux peuvent être tout matériau connu de l'homme du métier, par exemple un matériau choisi dans le groupe comprenant du papier absorbant, du coton, de la cellulose, de la fibre de verre, de la fibre de cellulose, une membrane de nitrocellulose. Il peut s'agir également des matériaux précités ayant subis un ou plusieurs traitement préalable, par exemple par mise en contact du matériau avec une solution. La solution peut comprendre un ou plusieurs composants chimiques par exemple du tétraborate de sodium et/ou du phosphate de sodium additionnés de détergeant comme du tween 20 et/ou du triton X-100 et/ou de protéines comme de l'albumine de sérum bovin ou de la caséine. La solution peut avoir un pH compris entre 6 et 10, de préférence entre 7 et 9. Avantageusement, la mise en contact de la solution avec le matériau peut permettre de modifier les propriétés de surface du matériau, par exemple le rendre hydrophile, améliorer la migration de l'échantillon, améliorer la fixation et la stabilité du conjugué, impacter les performances du test.

Avantageusement, la zone de dépôt (1) peut être choisie dans le groupe comprenant un papier absorbant, un support en fibre de verre et de la fibre de cellulose.

Avantageusement, la zone (2) comprenant des anticorps anti IGFBP-1 A, des anticorps anti IGFBP-1 B et des anticorps anti IL-6 marqués peut être de la fibre de verre et de la fibre de cellulose.

Avantageusement, les zones 1 et 2 peuvent être constituées du même matériau choisi choisie dans le groupe comprenant un papier absorbant, un support en fibre de verre et de la fibre de cellulose.

Avantageusement, les zones de révélation (3) à (5) peuvent être une membrane de nitrocellulose.

Les anticorps de capture dirigés contre l'IGFBP-1 A, dirigés contre l'IGFBP-1 B et dirigés contre l'IL-6 peuvent être, par exemple fixés directement sur le support de la zone, par exemple par couplage chimique et/ou par dépôt tel que décrit précédemment.

Lorsque es zones (3) à (5) sont situées sur un même support, les zones de fixation de l'anticorps de capture d'IGFBP-1A, d'IGFBP-1B et de l'IL6 sont préférentiellement différentes les unes des autres. Il peut s'agir par exemple de trois bandes parallèles comprenant chacune un anticorps de capture différent.

Les différentes zones (1) à (6) précitées peuvent être fixées sur un support solide, par exemple sur des cartes laminées, par exemple pouvant être découpées en bandelettes, et pouvant être comprises dans un contenant comprenant un ou plusieurs orifice(s) au niveau des zones (3) à (5) permettant une visualisation du résultat et un orifice au niveau de la zone (1) de dépôt de l'échantillon permettant le dépôt de l'échantillon.

Les différentes zones (1) à (6) précitées peuvent être fixées sur différents supports solides, par exemple sur des cartes laminées, par exemple en bandelettes et pouvant être comprises dans différents contenant. Par exemple, les différentes zones peuvent être fixées sur trois supports comprenant chacun une zone (1), une zone (2), une zone choisie dans le groupe comprenant les zones (3), (4) et (5) et une zone (6). Chaque support ou contenant peut comprendre un orifice au niveau des zones (3) à (5) permettant une visualisation du résultat et un orifice au niveau de la zone (1) de dépôt de l'échantillon permettant le dépôt de l'échantillon.

Le dispositif peut comprendre en outre une zone (6) d'absorption. Selon l'invention, la zone d'absorption (6) peut être n'importe quel support solide absorbant connu de l'homme du métier. Il peut s'agir, par exemple, d'un papier buvard absorbant, d'une éponge, d'un coton, d'une feutrine ou d'un textile synthétique.

Avantageusement, lorsque le dispositif comprend les différentes zones (1) à (5) précitées fixées sur un support solide, les supports des zones (1) et (2) se chevauchent à l'une de leur extrémité, l'autre extrémité de la zone (2) se chevauche avec une extrémité du matériau comprenant les zones (3) à (5). Le chevauchement des différentes zones (1) à (3-5) permet avantageusement lors de l'application du prélèvement et/ou de l'immersion de l'extrémité libre de la zone (1) dans le prélèvement la migration par capillarité du prélèvement dans les différentes zones. De préférence, les zones (1) et (2) sont disposées sur un même support. De préférence, la zone (6) d'absorption se chevauche avec l'extrémité libre de la zone (5). Ainsi, la zone (6) permet une migration accélérée du prélèvement à travers les différentes zones du dispositif. Avantageusement, la zone (6) permet d'absorber l'excédent de liquide du prélèvement.

Avantageusement, lorsque le dispositif comprend différentes zones pouvant être fixées sur trois supports comprenant chacun une zone (1), une zone (2) et une zone choisie dans le groupe comprenant les zones (3), (4) et (5). Sur un support les zones (1) et (2) se chevauchent à l'une de leur extrémité, l'autre extrémité de la zone (2) se chevauche avec une extrémité de la zone (3), sur un second support les zones (1) et (2) se chevauchent à l'une de leur extrémité, l'autre extrémité de la zone (2) se chevauche avec une extrémité de la zone (4), et sur un troisième support les zones (1) et (2) se chevauchent à l'une de leur extrémité, l'autre extrémité de la zone (2) se chevauche avec une extrémité de la zone (5). De préférence, une zone (6) d'absorption se chevauche indépendamment avec l'extrémité libre de la zone (3), (4) et/ou (5). Ainsi, la zone (6) permet une migration accélérée du prélèvement à travers les différentes zones du dispositif. Avantageusement, la zone (6) permet d'absorber l'excédent de liquide du prélèvement.

Le chevauchement des différentes zones (1) à (5) permet avantageusement lors de l'application du prélèvement et/ou de l'immersion de l'extrémité libre de la zone (1) dans le prélèvement la migration par capillarité du prélèvement dans les différentes zones.

Les différentes zones (1) à (6) peuvent être indépendamment recouvertes avec un film de protection. Il peut s'agir ; par exemple d'un film plastique, par exemple un film en chlorure de polyvinyle (PVC), un film biodégradable, par exemple un film en Polycaprolactone (PCL), en alcool Polyvinyle (PVA), en acide Polylactique (PLA).

Le film permet avantageusement de protéger indépendamment les différentes zones du dispositif de l'invention.

Le film peut être un film unique ou plusieurs films recouvrant indépendamment une des dites zones (1) à (6).

Le film ou les films peuvent recouvrir partiellement le dispositif de l'invention laissant ainsi certaines zones non couvertes. Selon l'invention, le film ou les films peut (peuvent) être un (des) film(s) détachable(s) qui peut (peuvent) être enlevé(s) avant l'utilisation du dispositif. Selon l'invention, le film peut comprendre une inscription à sa surface permettant avantageusement une identification du dispositif.

D'autres avantages pourront encore apparaître à l'homme du métier à la lecture des exemples ci-dessous, illustrés par les figures annexées, donnés à titre illustratif.

**Brève description des figures**

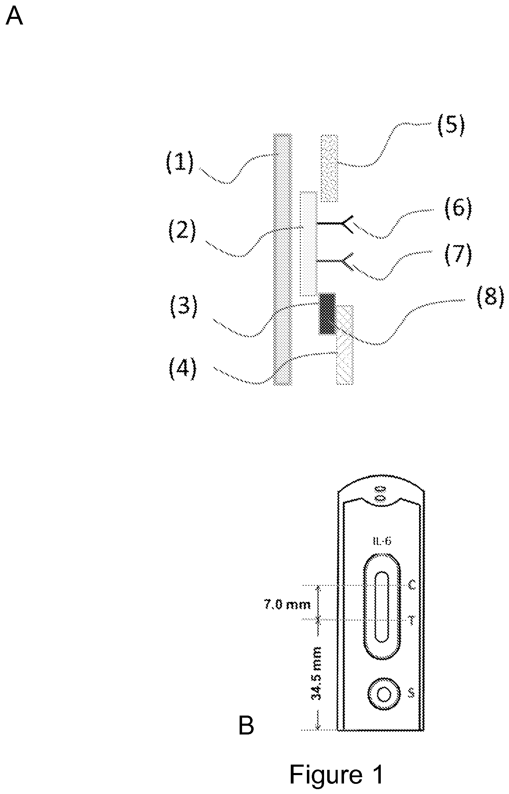

- La figure 1 A représente un schéma d'un dispositif de détection de l'interleukine 6 (IL-6) et le support B comprenant ledit dispositif. Sur cette figure (1) correspond à un support plastique avec adhésif de 6 cm de haut, (2) une membrane de nitrocellulose: 2,5 cm de hauteur, (3) un support de conjugué « Conjugate pad » commercialisé par la société Ahlstrom # 6613 traité; 1 cm, (4) un support pour échantillon « Sample pad »: commercialisé par la société Ahlstrom # 8964 traité; 1,6 cm, (5) un support d'absorption « Absorband pad »: commercialisé par la société Ahlstrom # 222; 1,6 cm, (6) un anticorps de chèvre anti IgG de souris, (7) un anticorps anti-IL6 humaine, (8) un anticorps marqué anti-IL6 humaine.

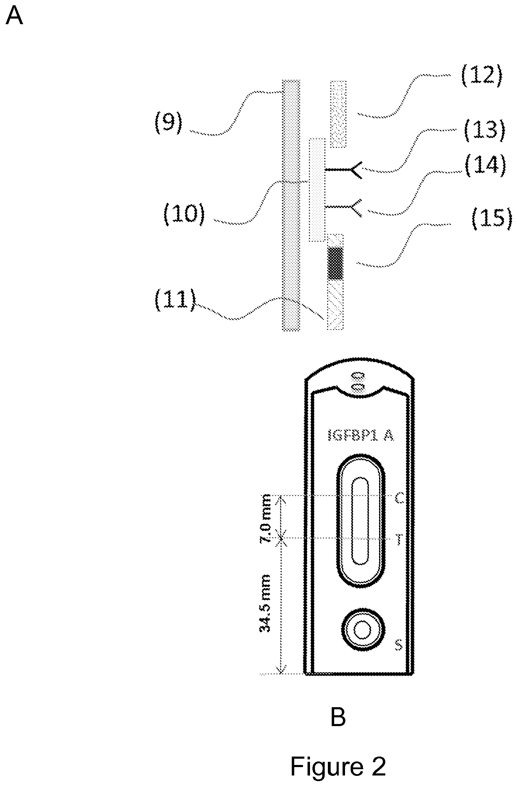

- La figure 2 A représente un schéma d'un dispositif de détection de l'IGFBP-1A et le support B comprenant ledit dispositif. Sur cette figure (9) correspond à un support plastique avec adhésif de 6 cm de haut, (10) une membrane de nitrocellulose: 2,5 cm de hauteur, (11) un support pour échantillon « Sample pad »: commercialisé par la société Ahlstrom # 8964 traité; 2,3 cm, (12) un support d'absorption « Absorband pad »: commercialisé par la société Ahlstrom # 222; 1,6 cm, (13) un anticorps de chèvre anti IgG de souris, (14) un anticorps de souris anti-IGFBP-1 humaine, (15) un anticorps marqué anti-IGFBP-1 humaine.

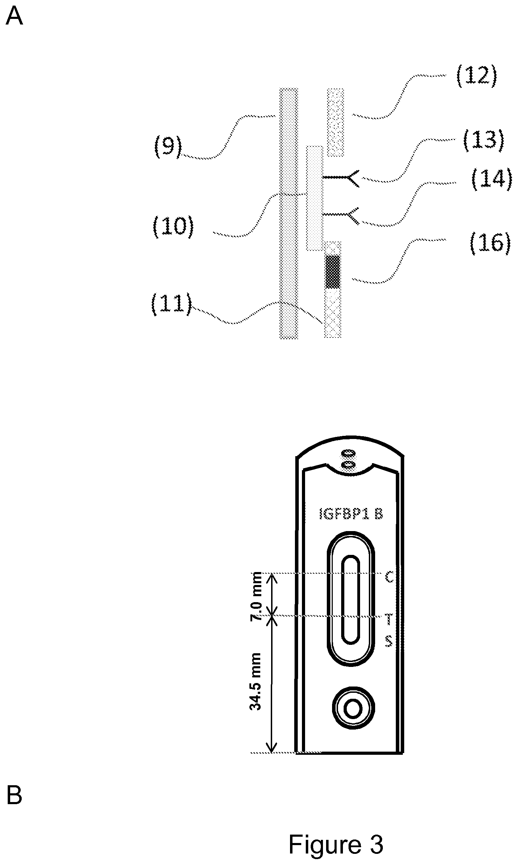

- La figure 3 A représente un schéma d'un dispositif de détection de l'IGFBP-1 B et le support B comprenant ledit dispositif. Sur cette figure (9) correspond à un support plastique avec adhésif de 6 cm de haut, (10) une membrane de nitrocellulose: 2,5 cm de hauteur, (11) un support pour échantillon « Sample pad »: commercialisé par la société Ahlstrom # 8964 traité; 2,3 cm, (12) un support d'absorption « Absorband pad »: commercialisé par la société Ahlstrom # 222; 1,6 cm, (13) un anticorps de chèvre anti IgG de souris, (14) un anticorps de souris anti-IGFBP-1 humaine, (16) un mélange d'anticorps marqué anti-IGFBP-1 N-terminale humaine et anti-IGFBP-1 humaine.

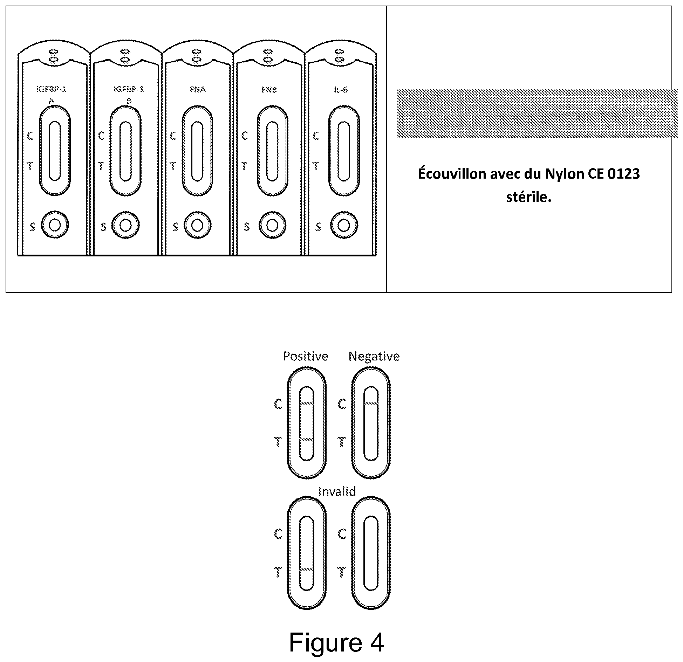

- La figure 4 représente un schéma d'un dispositif de détection de l'IL6, l'IGFBP-1A, l'IFBP-1B, la fibronectine A (FNA) et la fibronectine B (FNB) et l'écouvillon utilisé pour le prélèvement de l'échantillon.

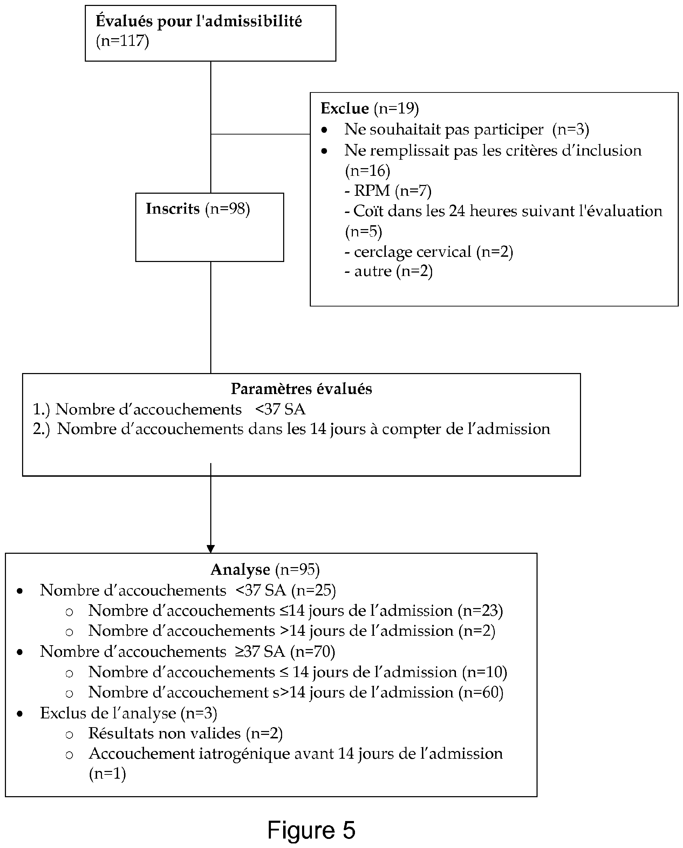

- La figure 5 représente un diagramme concernant l'étude de la prédiction détection d'accouchement prématuré.

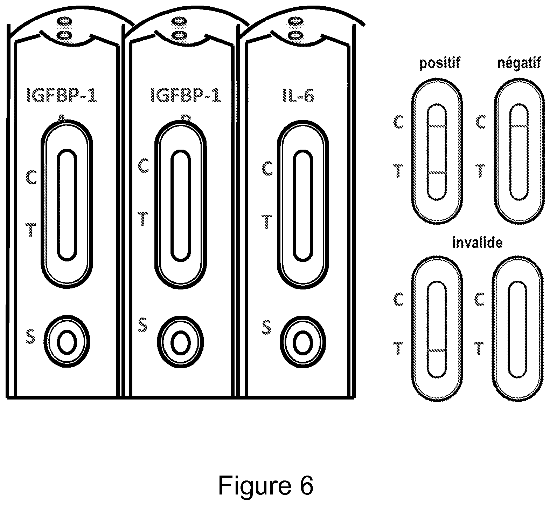

- La figure 6 représente le support comprenant des dispositifs de détection respectivement d'IL6, d'IGFBP-1A, d'IFBP-1B.

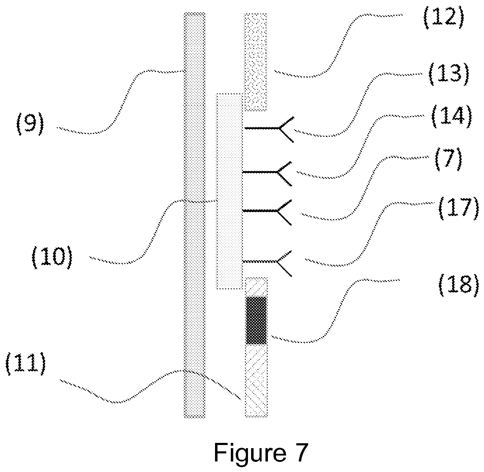

- La figure 7 représente un schéma d'un dispositif de détection d'IGFBP-1 A, d'IGFBP-1 B et d'IL-6. Sur cette figure (9) correspond à un support plastique avec adhésif de 6 cm de haut, (10) une membrane de nitrocellulose: 2,5 cm de hauteur, (11) un support pour échantillon « Sample pad »: commercialisé par la société Ahlstrom # 8964 traité; 2,3 cm, (12) un support d'absorption « Absorband pad »: commercialisé par la société Ahlstrom # 222; 1,6 cm, (13) un anticorps de chèvre anti IgG de souris, (14) un anticorps de souris anti-IGFBP-1 humaine, (7) un anticorps anti-IL6 humaine, (17) un anticorps anti- IGFBP-1 N-terminale humaine (18) un mélange d'anticorps marqués anti-IGFBP-1 humaine, et anti-IL(6) humaine.

**EXEMPLES**

**Exemple 1** : **Dispositif immunochromatographique pour évaluer le risque d'accouchement imminent**

Le dispositif est composé de trois bandelettes distinctes : une bandelette détectant l'IGFBP-1 A, une bandelette détectant l'IGFBP-1 B et une bandelette détectant l'IL-6.

Préparation de la bandelette IL-6

Les bandelettes ont été préparées à partir de cartes laminées de 30 cm X 6 cm (CNPC-SS12, MDI (marque déposée)) constituées d'un support en matière plastique recouvert d'une couche d'adhésif sur laquelle la membrane de nitrocellulose, le papier absorbant et les fibres de verre sont assemblés.

A l'aide d'un automate de distribution de réactifs (Isoflow Dispenser, Imagene Technology Inc. (marque déposée)), des anticorps de chèvre anti IgG de souris (ABGAM-0500, Arista Biologicals (marque déposée)) dilués à 0,5 mg/mL dans un tampon PBS ainsi que des anticorps anti IL-6 (numéro de catalogue 855.050.005, clone B-E8 Diaclone(marque déposée)) dilués à 1 mg/mL dans un tampon PBS ont été déposés avec un débit de 1 µl/cm sous forme de lignes parallèles espacées de 5 mm et d'une largeur de 1 à 2 mm chacune sur une membrane de nitrocellulose dont la largeur est de 25 mm.

Après dépôt de ces moyens de recherche, ces cartes ont été ensuite placées 30 minutes dans une étuve à 37°C sous atmosphère à taux d'humidité contrôlé inférieur à 30% pour les sécher.

Le mélange de conjugué, c'est-à-dire une solution comprenant des anticorps marqués, a été préparé à partir des anticorps anti IL-6 (numéro de catalogue 879.030.002, clone B-E4, Diaclone (marque déposée)) préalablement couplés à des particules d'or colloïdales selon le protocole suivant :

A 100mL d'eau distillée 1,0 mL de solution de chlorure d'or 1% a été ajouté (G4022, Sigma Aldrich). L'eau a été chauffée jusqu'au point d'ébullition et 2,5mL de solution de citrate de sodium 1% (S1804, Sigma Aldrich) ont été ajoutés. La solution est devenue incolore puis a viré au pourpre. Au bout d'une minute, la solution a été chauffée jusqu'à ce qu'elle devienne rouge cerise. 9,5mL de citrate de sodium 1% (S1804, Sigma Aldrich) ont été ajoutés. L'eau a été chauffée jusqu'au point d'ébullition et 8,6mL de solution de chlorure d'or 1% (G4022, Sigma Aldrich) ont été ajoutés. La solution est devenue bleue foncée quasi noire, puis pourpre puis a été laissée refroidir à température ambiante.

Le pH a été ajusté, à l'aide d'une solution de carbonate de potassium 0,2M (269619, Sigma Aldrich) de 10mL de la solution d'or colloïdal pourpre préalablement obtenue jusqu'à atteindre de pH 6,3. $200\mu g$ d'anti-IL-6 ont été ajoutés à 1mL d'eau distillée puis 10mL de solution d'or colloïdal pourpre au pH ajusté à 6,3 ont été ajoutés rapidement. Ce tube a été placé sur agitateur circulaire (Rotateur multidisques 5-50 T/min, SOBINCO (marque déposée)) pendant 20 minutes. 1,5 mL de Gold stabilization Buffer (Artron Bioresearch (marque déposée)) ont été ajoutés. Les 2 tubes ont été centrifugés (5804, Eppendorf (marque déposée)) à 4000 rcf pendant 30 minutes. Le surnageant a été aspiré à l'aide d'une pompe à vide (159600, BRAND (marque déposée)) et le culot a été repris avec 1mL de tampon de resuspension (pH 8,0) 20mM TRIS BASE (26-128-3094, Euromedex (marque déposée)), 50mM NaCl (S7653, Sigma Aldrich (marque déposée)), 0,2% BSA (1035-70, ID Bio (marque déposée)), 10% sucrose (S8501, Sigma Aldrich), 5% tréhalose (T9531, Sigma Aldrich (marque déposée)).

A $200\mu L$ de solution de conjugué IL-6 ont été ajoutés $800\mu L$ de la solution de saturation ((pH 7,4) 50mM $Na_2HPO_4$ (S7907, SIGMA ALDRICH (marque déposée)) 1% de d'Albumine de sérum de bovin (BSA) (1035-70, ID Bio), 20% de sucrose (S8501, SIGMA ALDRICH) et 5% de tréhalose (T9531, SIGMA ALDRICH (marque déposée)). Le mélange ainsi obtenu a été agité 20 minutes à température ambiante sur l'agitateur circulaire adapté aux tubes de 1,5 mL

Le mélange d'anticorps couplés aux particules d'or colloïdale a été ensuite vaporisé avec un débit de 4 $\mu l$/cm sur des feuilles de fibre de verre (zone conjuguate pad) de dimension 30 cm X 1 cm (# 6613 AHLSTROM, MAPDS-200, Arista Biologicals (marque déposée)) préalablement traitées avec une solution de 0,5% de Polyvinylalcool P8136, SIGMA ALDRICH (marque déposée)), 0,1% de triton X-100 (X-100, SIGMA ALDRICH (marque déposée)), 0,5% de d'Albumine de sérum de bovin (BSA) (1035-70, ID Bio), 50mM $Na_2HPO_4$ (S7907, SIGMA ALDRICH (marque déposée)) pH 7,4.

La fixation de ce mélange de conjugués sur la feuille de fibre de verre a été réalisée avec le même appareil Isoflow Dispenser (marque déposée) en se conformant aux indications du fabricant. Après vaporisation, les feuilles de fibre de verre sont séchées 30 minutes dans une étuve à 37°C sous atmosphère à taux d'humidité contrôlé inférieur à 30% pour les sécher.

L'assemblage des cartes a été fait par collage de la fibre de verre imprégnée de conjugué sur la partie adhésive inférieure de la carte en faisant chevaucher la feuille de fibre de verre de 1 à 3 mm sur la membrane de nitrocellulose. Un second papier de fibre de verre (zone Sample Pad) de dimension 30 cm X 1,6 cm (# 8964 AHLSTROM, MAPDS-0300, Arista Biologicals (marque déposée)) préalablement traitées avec une solution 0,1 M de $B_4Na_2O_7$ (B3545, SIGMA ALDRICH (marque déposée)) 1% triton X-100 (X-100, SIGMA ALDRICH (marque déposée)) pH 8,4 est collé dans la partie adhésive inférieure de la carte en chevauchant la feuille de fibre de verre imprégnée de conjugué de 1 à 3 mm.

De la même manière, un papier de haut pouvoir d'absorption (zone absorbant pad) (absorbant pad, Ahlstrom 222, MAPDS-0100, Arista Biologicals (marque déposée)) de dimension 30 cm X 1,6 cm a été placé sur la partie supérieure de la carte et chevauche la membrane de nitrocellulose de 1 à 3 mm afin de pouvoir créer un flux de migration.

La carte ainsi assemblée a été découpée en bandelettes de 4 mm de largeur à l'aide d'une guillotine (Modèle CM4000, BIODOT, (marque déposée))

Les bandelettes ont été ensuite déposées dans un support en plastique appelé cassette (MK001, D2 Technologies, (marque déposée))

Les cassettes ont été ensuite fermées à l'aide d'une clipseuse (Closure-I, A-Point Technologies, (marque déposée)).

La figure 1 représente une bandelette et un support comprenant ladite bandelette pour la détection d'IL-6.

Préparation de la bandelette IGFBP-1 A (IGFBP-1 intacte)

Les bandelettes ont été préparées à partir de cartes laminées de 30 cm X 6 cm (CNPC-SS12, MDI (marque déposée)) constituées d'un support en matière plastique recouvert d'une couche d'adhésif sur laquelle la membrane de nitrocellulose, le papier absorbant et la fibre de verre ont été assemblés.

A l'aide d'un automate de distribution de réactifs (Isoflow Dispenser, Imagene Technology Inc. (marque déposée)), des anticorps de chèvre anti IgG de souris (ABGAM-0500, Arista Biologicals (marque déposée)) dilués à 0,5 mg/mL dans un tampon PBS ainsi que des anticorps anti IGFBP-1 (numéro de catalogue 4IG8, clone C7B9, Hytest (marque déposée)) dilués à 1 mg/mL dans un tampon PBS ont été déposés avec un débit de 1 $\mu$l/cm sous forme de lignes parallèles espacées de 5 mm et d'une largeur de 1 à 2 mm chacune sur une membrane de nitrocellulose dont la largeur est de 25 mm.

Après dépôt de ces moyens de recherche, ces cartes ont été ensuite placées 30 minutes dans une étuve à 37°C sous atmosphère à taux d'humidité contrôlé inférieur à 30% pour les sécher.

Le mélange de conjugué, c'est-à-dire une solution comprenant des anticorps marqués, a été préparé à partir de l'anticorps anti IGFBP-1 (numéro de catalogue 4IG8, clone G5F8, Hytest (marque déposée)) préalablement couplé à des particules d'or colloïdales selon le protocole suivant :
A 100mL d'eau distillée 1,0 mL de solution de chlorure d'or 1% a été ajouté (G4022, Sigma Aldrich). L'eau a été chauffée jusqu'au point d'ébullition et 2,5mL de solution de citrate de sodium 1% (S1804, Sigma Aldrich) ont été ajoutés. La solution est devenue incolore puis a viré au pourpre. Au bout d'une minute, la solution a été chauffée jusqu'à ce qu'elle devienne rouge cerise. 9,5mL de citrate de sodium 1% (S1804, Sigma Aldrich) ont été ajoutés. L'eau a été chauffée jusqu'au point d'ébullition et 8,6mL de solution de chlorure d'or 1% (G4022, Sigma Aldrich) ont été ajoutés. La solution est devenue bleue foncée quasi noire, puis pourpre puis a été laissée refroidir à température ambiante.

Le pH a été ajusté, à l'aide d'une solution de carbonate de potassium 0,2M (269619, Sigma Aldrich) de 10mL de la solution d'or colloïdal pourpre préalablement obtenue jusqu'à atteindre de pH 7,8. 200$\mu$g d'anti-AIGFBP-1 clone F8 ont été ajoutés à 1mL d'eau distillée puis 10mL de solution d'or colloïdal pourpre au pH ajusté à 7,8 ont été ajoutés rapidement. Ces tubes ont été placés sur agitateur circulaire (Rotateur multidisques 5-50 T/min, SOBINCO (marque déposée)) pendant 20 minutes. 1,5mL de Gold Stabilization Buffer (Artron Bioresearch (marque déposée)) ont été ajoutés. Les 2 tubes ont été centrifugés (5804, Eppendorf (marque déposée)) à 4000 rcf pendant 30 minutes. Le surnageant a été aspiré à l'aide d'une pompe à vide (159600, BRAND (marque déposée)) et le culot a été repris avec 1mL de tampon de resuspension (pH 8,0) 20mM TRIS BASE (26-128-3094, Euromedex (marque déposée)), 50mM NaCl (S7653, Sigma Aldrich (marque déposée)), 0,2% BSA (1035-70, ID Bio (marque déposée)), 10% sucrose (S8501, Sigma Aldrich), 5% tréhalose (T9531, Sigma Aldrich (marque déposée)).

A 200 $\mu$L de solution de conjugué IGFBP-1 clone F8 ont été ajoutés 800 $\mu$L de la solution de saturation ((pH 7,4) 50mM $Na_2HPO_4$ (S7907, SIGMA ALDRICH (marque déposée)) 1% de d'Albumine de sérum de bovin (BSA) (1035-70, ID Bio), 20% de sucrose (S8501, SIGMA ALDRICH) et 5% de tréhalose (T9531, SIGMA ALDRICH (marque déposée))). Le mélange ainsi obtenu a été agité 20 minutes à température ambiante sur l'agitateur circulaire adapté aux tubes de 1,5 mL.

Le mélange d'anticorps couplés aux particules d'or colloïdale a été ensuite vaporisé avec un débit de 4 $\mu$l/cm sur des feuilles de fibre de verre (zone sample/conjuguate Pad) de dimension 30 cm X 3,2 cm (# 8964 AHLSTROM, MAPDS-0300, Arista Biologicals (marque déposée)) préalablement traitées avec une solution 0,1 M de $B_4Na_2O_7$ (B3545, SIGMA ALDRICH (marque déposée)) 1% triton X-100 (X-100, SIGMA ALDRICH (marque déposée)) pH 8,4.

La fixation de ce mélange de conjugués sur la feuille de fibre de verre a été réalisée avec le même appareil Isoflow Dispenser (marque déposée) en se conformant aux indications du fabricant. Après vaporisation, les feuilles de fibre de verre ont été séchées 30 minutes dans une étuve à 37°C sous atmosphère à taux d'humidité contrôlé inférieur à 30% pour les sécher.

L'assemblage des cartes a été fait par collage de la fibre de verre imprégnée de conjugué sur la partie adhésive inférieure de la carte en faisant chevaucher la feuille de fibre de verre de 1 à 3 mm sur la membrane de nitrocellulose. De la même manière, un papier de haut pouvoir d'absorption (zone absorbant pad) (Ahlstrom 222, MAPDS-0100, Arista Biologicals) de dimension 30 cm X 1,6 cm a été placé sur la partie supérieure de la carte et chevauche la membrane de nitrocellulose de 1 à 3 mm afin de pouvoir créer un flux de migration.

La carte ainsi assemblée a été découpée en bandelettes de 4 mm de largeur à l'aide d'une guillotine (Modèle CM4000, BIODOT.

Les bandelettes ont été ensuite déposées dans un support en plastique appelé cassette (MK001, D2 Technologies, (marque déposée))

Les cassettes ont été ensuite fermées à l'aide d'une clipseuse (Closure-I, A-Point Technologies, (marque déposée)).

La figure 2 représente une bandelette et un support comprenant ladite bandelette pour la détection d'IGFBP-1A.

Préparation de la bandelette IGFBP-1 B (IGFBP-1 totale)

Les bandelettes ont été préparées à partir de cartes laminées de 30 cm X 6 cm (CNPC-SS12, MDI (marque déposée)) constituées d'un support en matière plastique recouvert d'une couche d'adhésif sur laquelle la membrane de nitrocellulose, le papier absorbant et la fibre de verre ont été assemblés.

A l'aide d'un automate de distribution de réactifs (Isoflow Dispenser, Imagene Technology Inc. (marque déposée)), des anticorps de chèvre anti IgG de souris (ABGAM-0500, Arista Biologicals (marque déposée)) dilués à 0,5 mg/mL dans un tampon PBS ainsi que des anticorps anti IGFBP-1 (numéro de catalogue 4IG8, clone C7B9, Hytest (marque déposée)) dilués à 0,5 mg/mL dans un tampon PBS ont été déposés avec un débit de 1 $\mu$l/cm sous forme de lignes parallèles espacées de 5 mm et d'une largeur de 1 à 2 mm chacune sur une membrane de nitrocellulose dont la largeur est de 25 mm.

Après dépôt de ces moyens de recherche, ces cartes ont été ensuite placées 30 minutes dans une étuve à 37°C sous atmosphère à taux d'humidité contrôlé inférieur à 30% pour les sécher.

Le mélange de conjugué, c'est-à-dire une solution comprenant des anticorps marqués, ont été préparé à partir des anticorps anti IGFBP-1 (numéro de catalogue 4IG8, clone G5F8, Hytest (marque déposée)) et des anticorps anti IGFBP-1 (numéro de catalogue 4I52, clone G2, Hytest (marque déposée)) préalablement couplés à des particules d'or colloïdales selon le protocole suivant :

A 100mL d'eau distillée 1,0 mL de solution de chlorure d'or 1% a été ajouté (G4022, Sigma Aldrich). L'eau a été chauffée jusqu'au point d'ébullition et 2,5mL de solution de citrate de sodium 1% (S1804, Sigma Aldrich) ont été ajoutés. La solution est devenue incolore puis a viré au pourpre. Au bout d'une minute, la solution a été chauffée jusqu'à ce qu'elle devienne rouge cerise. 9,5mL de citrate de sodium 1% (S1804, Sigma Aldrich) ont été ajoutés. L'eau a été chauffée jusqu'au point d'ébullition et 8,6mL de solution de chlorure d'or 1% (G4022, Sigma Aldrich) ont été ajoutés. La solution est devenue bleue foncée quasi noire, puis pourpre puis a été laissée refroidir à température ambiante.

Le pH a été ajusté, d'une part, à l'aide d'une solution de carbonate de potassium 0,2M (269619, Sigma Aldrich) de 10mL de la solution d'or colloïdal pourpre préalablement obtenue jusqu'à atteindre de pH 8,8 et; d'autre part, ajusté à l'aide d'une solution de carbonate de potassium 0,2M (269619, Sigma Aldrich) de 10 mL de solution d'or colloïdal pourpre à pH 7,8. 200$\mu$g d'anti-IGFBP1 clone G2 ont été ajoutés à 1mL d'eau distillée puis 10mL de solution d'or colloïdal pourpre au pH ajusté à 8,8 ont été ajoutés rapidement. 200$\mu$g d'anti-AIGFBP-1 clone F8 ont été ajoutés à 1mL d'eau distillée puis 10mL de solution d'or colloïdal pourpre au pH ajusté à 7,8 ont été ajoutés rapidement. Ces tubes ont été placés sur agitateur circulaire (Rotateur multidisques 5-50 T/min, SOBINCO (marque déposée)) pendant 20 minutes. 1,5mL de solution Gold Stabilization Buffer (Artron Bioresearch (marque déposée)) ont été ajoutés. Les 2 tubes ont été centrifugés (5804, Eppendorf (marque déposée)) à 4000 rcf pendant 30 minutes. Le surnageant a été aspiré à l'aide d'une pompe à vide (159600, BRAND (marque déposée)) et le culot a été repris avec 1mL de tampon de resuspension (pH 8,0) 20mM TRIS BASE (26-128-3094, Euromedex (marque déposée)), 50mM NaCl (S7653, Sigma Aldrich (marque déposée)), 0,2% BSA (1035-70, ID Bio (marque déposée)), 10% sucrose (S8501, Sigma Aldrich), 5% tréhalose (T9531, Sigma Aldrich (marque déposée)).

A 125$\mu$L de solution de conjugué IGFBP-1 clone F8 additionnés de 50$\mu$L de conjugué IGFBP-1 clone G2 ont été ajoutés 825$\mu$L de la solution de saturation ((pH 7,4) 50mM $Na_2HPO_4$ (S7907, SIGMA ALDRICH (marque déposée)) 1% de d'Albumine de sérum de bovin (BSA) (1035-70, ID Bio), 20% de sucrose (S8501, SIGMA ALDRICH) et 5% de tréhalose (T9531, SIGMA ALDRICH (marque déposée))). Le mélange ainsi obtenu a été agité 20 minutes à température ambiante sur l'agitateur circulaire adapté aux tubes de 1,5 mL.

Le mélange d'anticorps couplés aux particules d'or colloïdale a été ensuite vaporisé avec un débit de 4 $\mu$l/cm sur des feuilles de fibre de verre (zone sample/conjuguate Pad) de dimension 30 cm X 3,2 cm (# 8964 AHLSTROM, MAPDS-0300, Arista Biologicals (marque déposée)) préalablement traitées avec une solution 0,1 M de $B_4Na_2O_7$ (B3545, SIGMA ALDRICH (marque déposée)) 1% triton X-100 (X-100, SIGMA ALDRICH (marque déposée)) pH 8,4.

La fixation de ce mélange de conjugués sur la feuille de fibre de verre a été réalisée avec le même appareil Isoflow Dispenser (marque déposée) en se conformant aux indications du fabricant. Après vaporisation, les feuilles de fibre de verre ont été séchées 30 minutes dans une étuve à 37°C sous atmosphère à taux d'humidité contrôlé inférieur à 30% pour les sécher.

L'assemblage des cartes a été faite par collage de la fibre de verre imprégnée de conjugué sur la partie adhésive inférieure de la carte en faisant chevaucher la feuille de fibre de verre de 1 à 3 mm sur la membrane de nitrocellulose. De la même manière, un papier de haut pouvoir d'absorption (zone absorbant pad) (Ahlstrom 222, MAPDS-0100, Arista Biologicals) de dimension 30 cm X 1,6 cm a été placé sur la partie supérieure de la carte et chevauchait la membrane de nitrocellulose de 1 à 3 mm afin de pouvoir créer un flux de migration.

La carte ainsi assemblée a été découpée en bandelettes de 4 mm de largeur à l'aide d'une guillotine (Modèle CM4000, BIODOT.

Les bandelettes ont été ensuite déposée dans un support en plastique appelé cassette (MK001, D2 Technologies, (marque déposée))

Les cassettes ont été ensuite fermées à l'aide d'une clipseuse (Closure-I, A-Point Technologies, (marque déposée)).

La figure 3 représente une bandelette et un support comprenant ladite bandelette pour la détection d'IGFBP-1B.

**Exemple 2 : Exemple d'utilisation d'un dispositif pour mettre en œuvre le procédé de l'invention**

1. Dans un flacon compte-goutte (015610, ALLTEST), une solution de 1 mL 50mM $Na_2HPO_4$(S7907, SIGMA AL-DRICH 1% d'Albumine de sérum de bovin (BSA) (1035-70, ID Bio) 0,05% triton X-100 (X-100, SIGMA ALDRICH (marque déposée)), 0,55% de tween 20, (P7949, SIGMA ALDRICH (marque déposée)), 0,1% de NaN3(S8032, SIGMA ALDRICH (marque déposée)) (pH 7,4) a été introduite,
2. l'échantillon a été prélevé avec un écouvillon stérile dont le bouton est du polyester puis déposé dans le flacon compte-goutte contenant la solution.
3. le mélange obtenu a été introduit dans le puits des cassettes décrites dans l'exemple 1
4. le résultat est obtenu après 10 minutes de migration

Le test est validé lorsque qu'une bande coloré apparait au niveau de la ligne contrôle

Le test est positif lorsqu'une bande colorée apparait au niveau d'au moins deux zones tests (IL-6, IGFBP-1A et/ou IGFBP-1 B)

**Exemple 3 : procédé de détection d'accouchement imminent**

Dans cet exemple, des femmes enceintes qui reçoivent des soins dans les cinq hôpitaux tertiaires dans le sud du Nigeria ont été incluses. L'étude a été approuvée par le Conseil institutionnel Examen des NAUTH (lettre d'approbation NAUTH / cs / 66 / VOL 8/56), et d'autres hôpitaux participants. Toute personne qui a accepté de participer à l'étude a donné un consentement éclairé.

Le diagnostic de risque d'accouchement imminent et de naissance prématurée a été réalisée en conformité avec le protocole des institutions, comprenant des femmes avec des membranes fœtales intactes, avec des contractions avec ou sans augmentation d'intensité et/ou de fréquence, un col partiellement ou totalement effacé, un col de l'utérus à moins de 3 cm. Les femmes enceintes avec un âge gestationnel de 24 + 0 à 36 + 6 présentant des symptômes ou des signes de risque de travail prématuré ont été incluses. Ces femmes présentaient des symptômes ou des signes autodéclarées, ou des plaintes suggestives du travail prématuré.

Pour l'admission, l'âge gestationnel établi sur la base de l'échographie du premier ou second trimestre devait être concordant avec les dates menstruelles. Les femmes pour lesquelles il y avait une divergence était notamment un dernière période menstruelle et l'âge gestationnel à l'échographie ≥ 10 jours à ≤20 semaines. Les critères d'exclusion étaient composés de grossesse multiple, polyhydramnios, la rupture prématurée de membranes fœtales, triplet, Examen cervicale antérieure, des rapports sexuels dans les 24 heures, une tocolyse, des saignements vaginaux, un cerclage du col utérin, un placenta praevia, ou une anomalie congénitale connue du fœtus, les femmes souffrant de maladies chroniques, (hypertension, le diabète, les maladies rénales ou cardiaques) ou d'anomalies du tractus génital ont été exclues. Les grossesses qui ont été accouchées moins de 14 jours post recrutement pour des indications maternelles et / ou fœtales ont également été exclues.

Suite à l'étude d'inclusion, les femmes ont eu un examen au spéculum effectué au cours de laquelle des échantillons de frottis vaginaux ont été recueillis pour mesurer/détecter l'IGFBP-1 totale, la fibronectine fœtale et de l'interleukine-6, préalablement à l'examen du col utérin. Les échantillons cervico-vaginales ont été recueillis au cours d'un examen au spéculum stérile à l'aide d'un écouvillon Dacron en plaçant l'extrémité inférieure de la tige de l'écouvillon dans le cul de sac postérieur du vagin pendant 30s (pour permettre la saturation du tampon). Après la collecte, le tampon a été inséré dans un tube contenant 1 ml de tampon d'extraction (voir figure 1). Les résultats ont été interprétés après attente pendant 10 minutes. Dans cet exemple les trois des marqueurs fibronectine, l'IGFBP-1 et l'interleukine-6 ont été étudiés. Pour ce faire, des anticorps monoclonaux / anticorps polyclonaux anti-fibronectine, l'IGFBP-1 et IL-6 anticorps couplé avec un marqueur bleu, un marqueur rose et des microsphères roses ont été utilisées.

Les anticorps utilisés étaient : pour l'IGFBP-1 A des anticorps anti IGFBP-1 commercialisés par la société Hytest (numéro de catalogue 4IG8 ; clone G5F8 et clone C7B9), pour l'IGFBP-1 B des anticorps commercialisés par la société Hytest numéro de catalogue 4I52 ; clone G2 ; numéro de catalogue 4IG8 ; clone G5F8 et clone C7B9, pour l'IL6 les anticorps commercialisés par Diaclone; numéro de catalogue 855.050.005, clone B-E8 ou B-E4. Les anticorps utilisés pour détecter la fibronectine (A et B) étaient des anticorps polyclonaux produits chez le lapin commercialisés par la société Sigma sous la référence F3648 (Sigma).

Pour la fibronectine et l'IGFBP1 deux formes différentes de ces protéines ont été utilisées comme marqueurs : la Fibronectine A et la Fibronectine B, et l'IGFBP-1A et l'IGFBP-1B. Après mesure, un suivi de l'accouchement ou de la possibilité d'accouchement ont été effectués 2, 7 et 14 jours après. Une comparaison des résultats via la détection des marqueurs: IGFBP-1A, IGFBP-1 B et-IL-6 avec la détection de la fibronectine A et B a été effectuée. Le test était considéré comme positif si au moins deux des trois biomarqueurs IGFBP-1, IGFBP-1 B et IL-6 étaient présent dans l'échantillon.

Les mesures cervicales ont été faites par échographie transvaginale. Au cours d'un examen gynécologique, l'examen au spéculum a été réalisé par introduction dans le vagin d'un speculum Cusco avant tout toucher vaginal. Les paramètres enregistrés à l'examen pelvien incluait la dilatation du col, l'état de la membrane, l'effacement du col utérin, les antécédents du patient, à savoir l'âge, le nombre de naissances et des fausses couches, et les résultats des tests de triplex.

Les examens cervicaux ont précédé chaque examen échographique. Pour minimiser la variabilité intra-observateur de la mesure du col de l'utérus, les femmes devaient vider leur vessie juste avant l'échographie et le canal cervical sagittale a été parfaitement identifié de telle sorte que l'orifice interne et l'orifice externe ont tous deux été identifiés et la longueur réelle mesurée correspondant à la distance correspondant à la droite entre eux.

Analyses statistiques

La taille de l'échantillon a été calculée comme étant de 94 avec une puissance de 80%, $\alpha = 0,05$, $\beta = 20\%$, et une valeur standard de l'effet de 0,84. Le test t de Student a été utilisé pour analyser les variables continues. La signification statistique des différences entre les groupes pour les variables continues a été évaluée et les différences de proportions ont été évaluées par le test de Mantel-Haenszel. Les données ont été analysées avec Epi Info Version 3.5.1 (Centers for Disease Control and Prévention, Atlanta, GA, USA) et la version 10 de Stata (StataCorp, College Station, TX, USA). Les valeurs de P <0,05 ont été considérées comme statistiquement significatives.

La sensibilité, spécificité, les valeurs prédictives positives et négatives et de précision ont également été déterminées. La sensibilité a été calculée comme suit: vrai positif (VP) / [VP + faux négatifs (FN)]; et la spécificité a été calculé comme suit: vrai négatif (VN) / [VN + faux positifs (FP)]. La valeur prédictive positive (VPP) = VP / (VP + FP), tandis que la Valeur Prédictive négative VPN = TN / (TN + FN). En outre, la précision = ([vrai positif + vrai négatif] / [vrai négatif + faux négatif + faux positif + vrai négatif] x 100%) de chaque test a également été estimée. La probabilité d'un accouchement prématuré a été mesurée en calculant le rapport de risque relatif (RR) ainsi que les intervalles de confiance respectifs (IC) à 95% pour chaque test.

117 femmes avec grossesses uniques ont été évalués pour l'admissibilité à participer à l'étude et 98 d'entre eux ont été recrutés. Sur les 98 femmes qui ont été recrutés, trois femmes ont été exclus (deux femmes avaient des résultats non valides et une femme ont subi une césarienne programmée en raison de facteur iatrogène avant les 14 jours la période de suivi et donc leurs données ont été exclus de l'analyse statistique). Un total de 95 femmes ont finalement été incluses dans l'analyse. Le modèle d'inclusion des femmes participant à l'étude est montré dans la figure 4.

Pour les femmes incluses, l'âge moyen était de 31,1 $\pm$ 2,3 ans (extrêmes: 20-40). La gravidité et la parité moyennes étaient respectivement de 2,5 $\pm$ 0,9 (gamme de 1-6) et 1,4 $\pm$ 0,4 (gamme de 0-5). L'âge gestationnel moyen à l'inclusion était de 34,1 $\pm$ 1,8 semaines (gamme de 24-36 semaines).

Les matrices de performance des tests (IGFBP-1 A, IGFBP-1 B et IL-6 par rapport à la fibronectine) dans les 2 jours, 7 jours et 14 jours d'inclusion dans l'étude sont présentées respectivement dans le Tableau 1, Tableau 2 et Tableau 3. Dans l'ensemble, l'exactitude prédictive des tests combinés par rapport aux jours d'inclusion était: 85,3% ($\leq$ 2 jours), 93,7% ($\leq$7 jours) et 94,7% ($\leq$ 14 jours) qui était plus élevée que la précision avec la fibronectine A (71,6%, 82,1% et 85,3%) et de la fibronectine B (57,9%, 70,5% et 71,6%) respectivement.

Sur les 95 patientes incluses dans l'analyse, 25 (26,3%) ont accouché avant 37 semaines de gestation et 70 (73,7%) ont accouchés à 37 semaines ou après de gestation. La matrice de la performance de l'IGFBP-1A / IGFBP-1B et IL-6 par rapport à la fibronectine en fonction du temps d'accouchement est décrite dans le tableau 4.

La température corporelle moyenne pour les femmes incluses dans l'étude était de 36,8 $\pm$ 0,4 ° C. La longueur moyenne du col échographique pour les femmes qui ont accouché dans les 14 jours suivant l'inclusion dans l'étude était de 24,4 $\pm$ 1,3 mm contre 32,5 $\pm$ 1,9 mm chez les femmes qui n'ont pas accouché dans les 14 jours suivant l'inscription. (T = -34,29; p <0,001).

Tableau 1 : résultats de l'association IGFBP-1A / IGFBP-1 B et IL-6 par rapport à la fibronectine (A / B) des tests chez les femmes en travail prématuré avec un risque d'accouchement à $\leq$ 2 jours après inclusion dans l'étude

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Vrai négatif | **62** | 51 | 38 | 68 | 61 | 52 |
| Vrai positif | **19** | 17 | 17 | 16 | 18 | 19 |

(suite)

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Faux négatif | **0** | 3 | 2 | 4 | 2 | 1 |
| Faux positif | **14** | 25 | 38 | 9 | 14 | 24 |
| Total | **95** | 95 | 95 | 95 | 95 | 95 |
| Spécificité % | **81,6** | 67,1 | 50 | 88,3 | 81,3 | 68,4 |
| Sensibilité | **100** | 85 | 89,5 | 80 | 90 | 95 |
| Valeur prédictive négative | **100** | 94,4 | 95 | 94,4 | 96,8 | 98,1 |
| Valeur prédictive positive | **57,6** | 40,5 | 30,9 | 64 | 56,3 | 44,2 |
| Précision | **85,3** | 71,6 | 57,9 | 88,4 | 83,2 | 74,7 |

La précision de 85,3% était statistiquement significative par rapport à la précision de la fibronectine A de 71,6% (Mantel-Haenszel chi carré = 5,23, p = 0,02, risque relatif = 1,59, IC à 95% = 01.02 à 02.25) ou de la fibronectine B de 57,9% (Mantel-Haenszel chi carré = 17,40, p <0,001, rapport de risque = 2,30, IC à 95% = 1,43 à 3,68).

Tableau 2 : résultats de l'association IGFBP-1A / IGFBP-1 B et IL-6 par rapport à la fibronectine (A / B) des tests chez les femmes en travail prématuré avec un risque d'accouchement à ≤ 7 jours après inclusion dans l'étude

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Vrai négatif | **60** | 50 | 38 | 63 | 60 | 50 |
| Vrai positif | **29** | 28 | 29 | 23 | 28 | 29 |
| Faux négatif | **4** | 3 | 2 | 8 | 2 | 2 |
| Faux positif | **4** | 14 | 26 | 1 | 5 | 14 |
| Total | **95** | 95 | 95 | 95 | 95 | 95 |
| Spécificité % | **93,8** | 78,1 | 59,4 | 98,4 | 96,8 | 78,1 |
| Sensibilité | **87,9** | 90,3 | 93,5 | 74,2 | 93,3 | 96,2 |
| Valeur prédictive négative | **93,8** | 94,3 | 95 | 88,7 | 96,8 | 96,2 |
| Valeur prédictive positive | **87,9** | 66,7 | 52,7 | 95,8 | 84,8 | 67,4 |

(suite)

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Précision | **93,7** | 82,1 | 70,5 | 90,5 | 92,6 | 83,2 |

La précision de 93,7% était statistiquement significative par rapport à la précision de la fibronectine A de 82,1% (Mantel-Haenszel chi carré = 5,95, p = 0,01, risque relatif = 2,04, IC à 95% = 1,01 à 4,12) ou de la fibronectine B de 70,5% (Mantel-Haenszel chi carré = 17,25, p <0,001, le rapport de risque = 3,23, IC à 95% = 1,54 à 6,77).

Tableau 3 : résultats de l'association IGFBP-1A / IGFBP-1 B et IL-6 par rapport à la fibronectine (A / B) des tests chez les femmes en travail prématuré avec un risque d'accouchement à ≤ 14 jours après inclusion dans l'étude

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Vrai négatif | **59** | 50 | 37 | 61 | 59 | 49 |
| Vrai positif | **31** | 31 | 31 | 23 | 31 | 31 |
| Faux négatif | **3** | 3 | 3 | 10 | 3 | 3 |
| Faux positif | **2** | 11 | 21 | 1 | 2 | 12 |
| Total | **95** | 95 | 95 | 95 | 95 | 95 |
| Spécificité % | **96,7** | 82 | 60,7 | 98,4 | 96,7 | 80,3 |
| Sensibilité | **94,2** | 91,2 | 91,2 | 69,7 | 91,2 | 94,2 |
| Valeur prédictive négative | **93,9** | 73,8 | 56,4 | 95,8 | 93,9 | 72,1 |
| Valeur prédictive positive | **93,9** | 73,8 | 56,4 | 95,8 | 93,9 | 72,1 |
| Précision | **94,7** | 85,3 | 71,6 | 88,4 | 94,7 | 84,2 |

La précision de 94,7% était statistiquement significative par rapport à la précision de la fibronectine A de 85,3% (Mantel-Haenszel chi carré = 4,71, p = 0,03, risque relatif = 2,00, IC à 95% = 0,93 à 4,30) ou de la fibronectine test B de 71,6% (Mantel-Haenszel chi carré = 18,09, p <0,001, le rapport de risque = 3,65, IC à 95% = 1,61 à 8,25).

Tableau 4: résultat de l'association par rapport au test de la fibronectine sur les cas de travail prématuré en fonction de l'âge gestationnel pour les femmes ayant accouché à ≤ 14 jours après inclusion dans l'étude

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Vrai négatif | **59** | 50 | 37 | 61 | 59 | 49 |

(suite)

| Marqueurs | 2/3 parmi IGFBP-1A/ IGFBP-1B & IL-6 | Fibronectine A (seuil de détection 500 ng/ml) | Fibronectine B (seuil de détection 50 ng/ml) | IGFBP-1A | IGFBP-1B | IL6 |
|---|---|---|---|---|---|---|
| Critères | | | | | | |
| Vrai positif | **31** | 31 | 31 | 23 | 31 | 31 |
| Faux négatif | **3** | 3 | 3 | 10 | 3 | 3 |
| Faux positif | **2** | 11 | 21 | 1 | 2 | 12 |
| Total | **95** | 95 | 95 | 95 | 95 | 95 |
| Spécificité % | **96,7** | 82 | 60,7 | 98,4 | 96,7 | 80,3 |
| Sensibilité | **91,2** | 91,2 | 91,2 | 69,7 | 91,2 | 91,2 |
| Valeur prédictive négative | **95,2** | 94,3 | 92,5 | 85,9 | 95,2 | 94,2 |
| Valeur prédictive positive | **93,9** | 73,8 | 56,4 | 95,8 | 93,9 | 72,1 |
| Précision | **94,7** | 85,3 | 71,6 | 88,4 | 94,7 | 84,2 |

Sur les 95 patients inclus dans l'analyse, 25 (26,3%) ont accouché avant 37 semaines de gestation et 70 (73,7%) ont accouchés à ou après 37 semaines de gestation. La matrice de la performance de l'IGFBP-1A/ IGFBP-1B et IL-6 par rapport à la fibronectine en fonction de la date d'accouchement est montré dans le tableau 2.

La température corporelle moyenne pour les femmes était de 36,8 $\pm$ 0,4°C. La longueur moyenne du col échographique pour les femmes qui ont accouchées dans les 14 jours suivant l'inclusion était de 24,4 $\pm$ 1,3 mm vs 32,5 $\pm$ 1,9 mm chez les femmes qui n'ont pas accouchées dans les 14 jours suivant l'inscription. (T = -34,29; p <0,001).

Tel que démontré dans cet exemple, le procédé de l'invention dans lequel au moins deux des marqueurs sont positifs permet l'obtention de résultats plus sensible, plus spécifique avec une meilleure valeur prédictive positive et négative que le test de référence connu utilisant la fibronectine [13]. En particulier, le procédé selon l'invention permet l'obtention de résultats avec une valeur prédictive positive très supérieur à celui utilisant la fibronectine.

En particulier, tel que démontré dans cet exemple, le procédé de l'invention permet l'obtention d'une forte sensibilité de 91,2%, avec une précision, la spécificité, la valeur prédictive positive et la valeur prédictive négative de test combiné plus élevé, par exemple 25% supérieure, que pour la fibronectine A ou fibronectine B respectivement. Les différences de résultats étaient statistiquement significatives (p <0,05).

En outre, tel que démontré dans cet exemple, le procédé permet l'obtention d'une sensibilité égale à 100% et une valeur prédictive négative de 100 % pour une menace d'accouchement à deux jours.

Tel que démontré dans cet exemple, le procédé selon l'invention permet, de par sa sensibilité, spécificité, valeur prédictive négative et positive permet d'identifier avec précision les femmes qui auront travail prématuré spontané. En effet, la sensibilité est de 91,2% et la spécificité est de 96,7. La valeur prédictive négative élevée, 93,9%, combiné avec la forte sensibilité et spécificité permet d'éviter les interventions médico-obstétrique inutiles dans la grossesse.

En outre, dans le cas de grossesse à terme, la sensibilité et la spécificité était respectivement de 81,8% et 96,6% démontrant clairement que le procédé selon l'invention est un procédé utile pour la prédiction d'une grossesse à terme.

En outre, une analyse des résultats lorsque les trois marqueurs sont positifs et/ou négatifs en fonction de la date de l'accouchement a été réalisée et sont présentés dans le tableau 5 ci-dessous.

Tableau 5: résultat du test pour les femmes ayant accouché ou non à moins de 14, 7 et 2 jours après inclusion dans l'étude

| | | Accouchement < 14 jours | | |
|---|---|---|---|---|
| | | non | oui | Total |
| IGFBP-1 A + | Triple négatif | 48 | 1 | 49 |
| | Triple positif | 1 | 23 | 24 |
| | Autre résultat | 12 | 10 | 22 |
| IGFBP-1 B + IL-6 | Total | 61 | 34 | 95 |

| | | Accouchement < 7 jours | | |
|---|---|---|---|---|
| | | Non | oui | Total |
| IGFBP-1 A + IGFBP-1 B + IL-6 | Triple négatif | 48 | 1 | 49 |
| | Triple positif | 1 | 23 | 24 |
| | Autre résultat | 16 | 6 | 22 |
| | Total | 65 | 30 | 95 |

| | | Accouchement < 2jours | | |
|---|---|---|---|---|
| | | Non | Oui | Total |
| IGFBP-1 A + IGFBP-1 B + IL-6 | Triple négatif | 49 | 0 | 49 |
| | Triple positif | 14 | 10 | 24 |
| | Autre résultat | 19 | 3 | 22 |
| | Total | 82 | 13 | 95 |

| Résultats avec un résultat triple positif ou triple négatif | | Accouchement | | |
|---|---|---|---|---|
| | | <14 jours | < 7jours | < 2jours |
| IGFBP-1 A + IGFBP-1 B + IL-6 | Sensibilité | 95,8% | 95,8% | 100,0% |
| | Spécificité | 98,0% | 98,0% | 77,8% |
| | VPN | 98,0% | 98,0% | 100,0% |
| | VPP | 95,8% | 95,8% | 41,7% |
| | Autre résultat | 23,2% | 23,2% | 23,2% |

Tel que démontré ci-dessous, le procédé de l'invention permet avantageusement d'obtenir un résultat avec une sensibilité et une spécificité égale respectivement à 95,8% et 98,0% pour une menace d'accouchement à 7 ou 14 jours.

En outre, lorsque le résultat et triple négatif, la valeur prédictive négative pour un accouchement à 7 ou 14 jours est égale à 98%, et de 100% pour un accouchement à 2 jours.

Ainsi, cet exemple démontre clairement que la détermination de la présence ou de l'absence dans les sécrétions vaginales d'au moins deux marqueurs choisi parmi l'IGFBP-1 (A et B) et l'IL-6 est un procédé fiable pour la prédiction d'accouchement imminent chez les femmes en travail prématuré et présente une sensibilité et une spécificité pour la prédiction d'accouchement imminent très supérieur aux procédés de références utilisés en clinique. En outre, cet exemple démontre clairement que l'utilisation des IGFBP-1 et de l'IL-6 permet de maximiser la sensibilité et de minimiser les diagnostics faux positifs. De plus, le procédé selon l'invention permet l'obtention de « vrai » négatif avec une valeur prédictive négative égale à 98% ou 100% permettant ainsi d'éviter des interventions cliniques et/ou des traitements pharmacologiques inutiles.

**Exemple 4 : procédé de détection d'accouchement imminent**

Dans cet exemple, les protocoles d'admission, de réalisation du test, d'examens après inclusion et les femmes incluses étaient identiques à ceux de l'exemple 3 ci-dessous.

La recherche de l'IGFBP-1 A, l'IGFBP-1 B et l'IL6 a été effectuée comme décrit dans l'exemple 3 ci-dessus.

Lorsque l'IGFBP-1 A été détectée une valeur $I_A$ égale à 2 a été attribuée, lorsque l'IGFBP-1 B a été détectée une valeur $I_B$ égale à 2 a été attribuée et lorsque l'IL-6 a été détectée une valeur $I_6$ égale à 1 a été attribuée. En l'absence de détection la valeur attribuée était de 0 pour chacun des marqueurs.

Après affectation des valeurs $I_A$; $I_b$, et $I_6$ une sommation des valeurs a été effectuée selon le score $S_1$ avec la formule suivante :

$$S_1 = I_A + I_B + I_6$$

Les valeurs du score $S_1$ obtenu pour les femmes ayant accouchées ou non à moins de 14, 7 et 2 jours après inclusion dans l'étude sont résumés dans le tableau 6 ci-dessous.

Tableau 6: résultat du test pour les femmes ayant accouché ou non à moins de 14, 7 et 2 jours après inclusion dans l'étude

| Score $S_1$ Total | Nombre de patientes | Nombre de patientes ayant accouché | | |
|---|---|---|---|---|
| | | sous 2 jours | entre 3-7 jours | entre 8-14 jours |
| 0 | 50 | 0 | 1 | 0 |
| 1 | 12 | 1 | 0 | 0 |
| 2-4 | 11 | 2 | 4 | 4 |
| 5 | 24 | 16 | 7 | 0 |

A partir des résultats décrits dans le tableau 6 ci-dessus, les probabilités calculées d'accoucher ont été calculés à partir des résultats obtenus en fonction du score obtenu sont résumés dans le tableau 7 ci-dessous.

Tableau 7: Probabilité d'accoucher en fonction du score

| Score $S_1$ | Nombre de patientes | Probabilité d'accoucher | | |
|---|---|---|---|---|
| | | sous 2 jours | sous 7 jours | sous 14 jours |
| 0 | 50 | 0% | 2% (1/50) | 2% (1/50) |
| 1 | 12 | 8,30% (1/12) | 8,30% (1/12) | 8,30% (1/12) |
| 2-4 | 11 | 18,20% (2/11) | 54,50% (6/11) | 90,90% (10/11) |
| 5 | 24 | 66,70% (16/24) | 95,80% (23/24) | 95,80% (23/24) |

Tel qu'illustré dans le tableau 7 ci-dessus un score S1 supérieur ou égal à 2 implique une probabilité d'accoucher d'ici à 14 jours supérieure à 90,90 %.

Les valeurs valeur prédictive négative et positive ont été calculées selon les formules suivantes :

$$VPP = VP/(VP+FP) \text{ et } VPN = VN/(VN+FN)$$

dans lesquels VP signifie Vrai Positif, FP signifie Faux Positif, VN signifie Vrai négatif et FN signifie Faux négatif.

En outre, une évaluation de la valeur prédictive négative et positive lorsque le score S1 obtenu était supérieur ou égale à 2 en fonction du délai d'accouchement, inférieur ou égal à 2 jours, à 7 jours ou à 14 jours a été effectuée. Le tableau 8 ci-dessous résume les résultats obtenus.

Tableau 8 : valeurs prédictive négative ou positive avec un score supérieur ou égal à 2.

| Score S$_1$ ≥ 2 | Délai d'accouchement | | |
|---|---|---|---|
| | Inférieur ou égale à 2 jours | Inférieur ou égale à 7 jours | Inférieur ou égal à 14 jours |
| Valeur Prédictive Positive (VPP) | 51,4% | 82,9% | 94,3% |
| Valeur Prédictive négative (VPN) | 98,4% | 96,8% | 96,8% |

Tels que démontré dans le tableau 8 ci-dessus, quel que soit l'imminence de l'accouchement, par exemple inférieur à 2 jours, inférieur à 7 jours et/ou inférieur à 14 jours, lorsque le score S$_1$ est supérieur ou égal à 2, la valeur prédictive négative est supérieure à 96,8% allant jusqu'à 98,4%.

Une évaluation de la valeur prédictive négative lorsque le score S1 obtenu était égal à 0 ou de la valeur prédictive positive lorsque le score S1 obtenu était égal à 5 en fonction du délai d'accouchement, inférieur ou égal à 2 jours, à 7 jours ou à 14 jours a été effectuée. Le tableau 9 ci-dessous résume les résultats obtenus.

Tableau 9 : valeur prédictive négative avec un score égal à 0 et valeur prédictive positive avec un score égal à 5.

| Score S$_1$ | | Délai d'accouchement | | |
|---|---|---|---|---|
| | | ≤ 2 jours | ≤ 7 jours | ≤ 14 jours |
| 5 | VPP | 66,7% | 95,8% | 95,8% |
| 0 | VPN | 100,0% | 98,0% | 98,0% |

En outre, tel que démontré dans le tableau 9 ci-dessus, le procédé permet l'obtention d'une valeur prédictive négative de 100 % pour une menace d'accouchement à deux jours et égale à 98% d'ici à 14 jours.

L'exemple est également réalisé avec différente affectation de valeurs pour chacun des marqueurs l'IGFBP-1 A, l'IGFBP-1 B et l'IL6. Le tableau 10 ci-dessous résume les différentes affectations, valeurs discriminantes du score et résultats associés.

Tableau 10 : affectation des valeurs et calcul des scores correspondants

| Marqueurs | Présent | Absent | S$_1$= I$_A$+ I$_B$+I$_6$ | | Délai d'accouchement | | |
|---|---|---|---|---|---|---|---|
| | | | | | Inférieur ou égale à 2 jours | Inférieur ou égale à 7 jours | Inférieur ou égal à 14 jours |
| IGFBP-1 A I$_A$ | 2 | 0 | S1 = 0 | VPN | 100,0% | 98,0% | 98,0% |
| IGFBP-1 B I$_B$ | 2 | 0 | S$_1$ ≥ 2 | VPP | 51,4% | 82,9% | 94,3% |
| | | | S$_1$ < 2 | VPN | 98,4% | 96,8% | 96,8% |
| IL6 I$_6$ | 1 | 0 | S1 = 5 | VPP | 66,7% | 95,8% | 95,8% |

(suite)

| Marqueurs | Présent | Absent | | $S_1 = I_A + I_B + I_6$ | | ≤ 2 jours | ≤ 7 jours | ≤ 14 jours |
|---|---|---|---|---|---|---|---|---|
| IGFBP-1 A $I_A$ | 3 | 0 | | S1 = 0 | VPN | 100,0% | 98,0% | 98,0% |
| IGFBP-1 B $I_B$ | 2 | 0 | | $S_1 \geq 2$ | VPP | 51,4% | 82,9% | 94,3% |
| | | | | $S_1 < 2$ | VPN | 98,4% | 96,8% | 96,8% |
| IL6 $I_6$ | 1 | 0 | | S1 = 6 | VPP | 66,7% | 95,8% | 95,8% |
| IGFBP-1 A $I_A$ | 3 | 0 | | S1 = 0 | VPN | 100,0% | 98,0% | 98,0% |
| IGFBP-1 B $I_B$ | 3 | 0 | | $S_1 \geq 3$ | VPP | 51,4% | 82,9% | 94,3% |
| | | | | $S_1 < 3$ | VPN | 98,4% | 96,8% | 96,8% |
| IL6 $I_6$ | 1 | 0 | | S1 = 7 | VPP | 66,7% | 95,8% | 95,8% |
| Marqueurs | Présent | Absent | | $S_1 = I_A + I_B + I_6$ | | ≤ 2 jours | ≤ 7 jours | ≤ 14 jours |
| IGFBP-1 A $I_A$ | 4 | 0 | | S1 = 0 | VPN | 100,0% | 98,0% | 98,0% |
| IGFBP-1 B $I_B$ | 3 | 0 | | $S_1 \geq 3$ | VPP | 51,4% | 82,9% | 94,3% |
| | | | | $S_1 < 3$ | VPN | 98,4% | 96,8% | 96,8% |
| IL6 $I_6$ | 1 | 0 | | S1 = 8 | VPP | 66,7% | 95,8% | 95,8% |

Tel que démontré dans cet exemple, le procédé selon l'invention permet, de par sa sensibilité, spécificité, valeur prédictive négative et positive identifie avec précision les femmes qui accoucheront dans un délai prédéfini.

En outre, de la valeur prédictive négative très élevée, à savoir supérieure à 98% voire égal à 100%, le procédé selon l'invention permet de réduire les résultats faux négatifs et fournis donc un résultat fiable et utilisable par les cliniciens.

Tel que démontré dans cet exemple, le procédé selon l'invention permet, de par sa sensibilité, spécificité, valeur prédictive négative et positive identifie avec précision les femmes qui accoucheront dans un délai prédéfini permettant d'éviter des hospitalisations et des interventions médico-obstétrique inutiles dans la grossesse.

Ainsi, cet exemple démontre clairement que le procédé comprenant le calcul du score $S_1$ selon l'invention est un procédé fiable pour la prédiction d'accouchement imminent chez les femmes en travail prématuré et présente une sensibilité et une spécificité pour la prédiction d'accouchement imminent très supérieur aux procédés de références utilisés en clinique.

De plus, cet exemple démontre clairement qu'un procédé selon l'invention permet l'obtention de « vrai » négatif avec une valeur prédictive négative égale à 98% ou 100% permettant ainsi d'éviter des hospitalisations, des interventions cliniques et/ou des traitements pharmacologiques inutiles.

**Listes de références**

1. Eroglu D, Yanik F, Oktem M, Zeyneloglu HB, Kuscu E. Prediction of preterm delivery among women with threatened preterm labor. 2007;64(2):109-16. Epub 2007 Feb 23.

2. Berghella V. Novel developments on cervical length screening and progesterone for preventing preterm birth. BJOG. 2009 Jan;116(2):182-7. doi: 10.1111/j.1471-0528.2008.02008.x.

3. Berghella V, Baxter JK, Hendrix NW. Cervical assessment by ultrasound for preventing preterm delivery. Cochrane Database Syst Rev. 2013 Jan 31;1:CD007235. doi: 10.1002/14651858.CD007235.pub3.

4. Goepfert AR, Goldenberg RL, Andrews WW, Hauth JC, Mercer B, et al. The Preterm Prédiction Study: association between cervical interleukin 6 concentration and spontaneous preterm birth. National Institute of Child Health and Human Development Maternal-Fetal Medicine Units Network. Am J Obstet Gynecol. 2001 Feb;184(3):483-8.

5. Leitich H, Egarter C, Kaider A, Hohlagschwandtner M, Berghammer P, Husslein P. Cervicovaginal fetal fibronectin as a marker for preterm delivery: a meta-analysis. Am J Obstet Gynecol. 1999 May;180(5):1169-76.

6. Goldenberg RL, Mercer BM, Meis PJ, Copper RL, Das A, McNellis D. The preterm prédiction study: fetal fibronectin testing and spontaneous preterm birth. NICHD Maternai Fetal Medicine Units Network. Obstet Gynecol. 1996 May;87(5 Pt 1):643-8.

7. McLaren JS, Hezelgrave NL, Ayubi H, Seed PT, Shennan AH. Prediction of spontaneous preterm birth using quantitative fetal fibronectin after recent sexual intercourse. Am J Obstet Gynecol. 2015 Jan; 212(1):89.e1-5. Epub 2014 Jun 30.

8. Kurkinen-Räty M, Ruokonen A, Vuopala S, Koskela M, Rutanen EM, Kärkkäinen T, Jouppila P. Combination of cervical interleukin-6 and -8, phosphorylated insulin-like growth factor-binding protein-1 and transvaginal cervical ultrasonography in assessment of the risk of preterm birth. BJOG. 2001 Aug;108(8):875-81.

9. Rutanen et al, Biochem Biophys Res Commun 1988 ; 152 : 208

10. Pekonen et al, J immunoassay 1989 ; 10 : 325-337

11. Beer et al. Qualification of cellulose nitrate membranes for lateral-flow assays, IVD technology, janvier 2002

12. Anne Harwood Peruski et al. Immunological Methods for Détection and Identification of Infectious Disease and Biological Warfare Agents. Clinical and diagnostic laboratory immunology, July 2003, p. 506-513

13. Honest H, Forbes CA, Duree KH, et al. Screening to prevent spontaneous preterm birth: systematic reviews of accuracy and effectiveness literature with economic modelling. Health Technol Assess 2009;13:1-627.

14. Riboni et al., Combination of biochemical markers in predicting pre-term delivery. Archives of Gynecology and Obstetrics 2011; 285(1): 61-66.

## Revendications

1. Procédé de détection/prédiction *in-vitro* d'un accouchement imminent, comprenant une étape de recherche simultanée, dans un prélèvement de sécrétions vaginales ou cervicales, des marqueurs du groupe constitué de la protéine 1 de liaison au facteur de croissance Insulinomimétique intacte (IGFBP-1 intacte), de la protéine 1 de liaison au facteur de croissance Insulinomimétique totale comprenant la protéine 1 de liaison au facteur de croissance Insulinomimétique intacte et un fragment N-Terminal de la protéine 1 de liaison au facteur de croissance Insulinomimétique (IGFBP-1 totale) et de l'interleukine 6 (IL-6), un accouchement imminent est détecté/prédit lorsque au moins deux des marqueurs du groupe constitué de la protéine 1 de liaison au facteur de croissance Insulinomimétique intacte (IGFBP-1 intacte), de la protéine 1 de liaison au facteur de croissance Insulinomimétique totale (IGFBP-1 totale et de l'interleukine 6 (IL-6), sont détectés.

2. Procédé de détection/prédiction in-vitro d'un accouchement imminent dans un échantillon de sécrétions vaginales ou cervicales comprenant :

   a) Recherche de la présence d'IGFBP-1 intacte, d'IGFBP-1 totale et d'IL-6
   b) Affectation d'une valeur d'IGFBP-1 intacte ($I_A$) si l'IGFBP-1 intacte est présente dans ledit échantillon ou de 0 si l'IGFBP-1 intacte n'est pas présente,

c) Affectation d'une valeur d'IGFBP1 B ($I_B$) si l'IGFBP-1 totale est présente dans ledit échantillon ou de 0 si l'IGFBP-1 totale n'est pas présente, la valeur d'$I_B$ affectée si l'IGFBP-1 totale est présente dans ledit échantillon étant inférieure ou égale à la valeur d'$I_A$ affectée lorsque l'IGFBP-1 intacte est présente dans ledit échantillon

d) Affectation d'une valeur d'IL-6 ($I_6$) si l'IL-6 est présente dans ledit échantillon ou de 0 si l'IL-6 n'est pas présente, la valeur $I_6$ affectée lorsqu'IL-6 est présente dans ledit échantillon étant strictement inférieure aux valeurs d'$I_A$ et $I_B$ affectées lorsque l'IGFBP-1 intacte et l'IGFBP-1 totale sont respectivement présentes dans ledit échantillon,

e) Calcul du score S1 selon la formule suivante :

$$S_1 = I_A + I_B + I_6$$

une valeur de S1 supérieure ou égale à la valeur d'$I_A$ ou $I_B$, respectivement affectée lorsque l'IGFBP-1 intacte ou l'IGFBP-1 totale est présente dans ledit échantillon, indiquant un accouchement imminent.

3. Procédé selon la revendication 1 ou 2 dans lequel la recherche est effectuée avec au moins un anticorps de capture dirigé contre l'IL-6, au moins un anticorps de capture dirigé contre l'IGFBP-1 intacte et au moins un anticorps de capture dirigé contre l'IGFBP-1 totale.

4. Procédé selon la revendication 2 ou 3 dans lequel la recherche est effectuée à partir d'un échantillon de sécrétions vaginales ou cervicales dilué dans une solution tampon.

5. Procédé selon l'une quelconque des revendications 1 ou 3 dans lequel la recherche est effectuée à partir d'un prélèvement de sécrétions vaginales ou cervicales dilué dans une solution tampon.

6. Procédé selon l'une quelconque des revendications précédentes lorsqu'elles dépendent de la revendication 1 dans lequel le procédé comprend l'ajustement d'au moins un des paramètres choisis parmi le volume, le pH, la force ionique du milieu tampon.

7. Procédé selon la revendication 6 dans lequel le procédé comprend l'ajustement d'au moins un des paramètres choisis parmi la concentration des anticorps de capture, ledit ajustement étant spécifique à l'IL-6, à l'IGFBP-1 intacte ou à l'IGFBP-1 totale.

8. Procédé selon la revendication 6 ou 7 dans lequel le procédé comprend l'ajustement d'au moins un des paramètres choisis parmi la concentration des anticorps de capture, ledit ajustement étant spécifique à l'IL-6, à l'IGFBP-1 intacte et/ou l'IGFBP-1 totale.

9. Procédé selon l'une quelconque des revendications 6 à 8 dans lequel le pH de la solution tampon est de 5 à 10.

10. Procédé selon l'une quelconque des revendications précédentes dans lequel ladite étape de recherche comprend une étape de révélation effectuée avec au moins un anticorps marqué anti IGFBP-1 intacte, au moins un anticorps marqué anti IGFBP-1 totale et au moins un anticorps marqué anti IL-6.

11. Procédé selon l'une quelconque des revendications précédentes, dans lequel l'IGFBP-1 intacte est détectée à partir d'une concentration de 40 ng/ml.

12. Procédé selon l'une quelconque des revendications précédentes, dans lequel l'IGFBP-1 totale est détectée à partir d'une concentration de 20 ng/mL intacte et/ou de 10 ng/ml du fragment N-Ter d'IGFBP-1.

13. Procédé selon l'une quelconque des revendications précédentes, dans lequel l'IL-6 est détectée à partir d'une concentration de 0,25 ng/ml.

14. Dispositif de mise en œuvre du procédé selon l'une quelconque des revendications 1 à 13 comprenant:

- une zone (1) de dépôt d'un échantillon
- une zone (2) comprenant des anticorps anti IGFBP-1 intacte, des anticorps anti IGFBP-1 totale et des anticorps anti IL-6 marqués.

- une zone (3) de révélation comprenant des anticorps de capture dirigés contre IGFBP-1 intacte,
- une zone (4) de révélation comprenant des anticorps de capture dirigés contre l'IGFBP-1 totale,
- une zone (5) de révélation comprenant des anticorps de capture dirigés contre IL-6.

15. Dispositif selon la revendication 14 dans lequel la zone de dépôt (1) est choisie dans le groupe comprenant un papier absorbant et un support en fibre de verre.

16. Dispositif selon la revendication 14 ou 15 dans lequel la zone de révélation (4) et/ou (5) est une membrane de nitrocellulose.

17. Dispositif selon l'une quelconque des revendications 14 à 16 dans lequel la zone (2) et/ou (3) est un support en fibre de verre.

18. Dispositif selon l'une quelconque des revendications 14 à 17 dans lequel les zones (1), (2) et/ou (3) est un support en fibre de verre ou de nitrocellulose.

19. Utilisation du dispositif selon l'une quelconque des revendications 14 à 18 pour la mise en œuvre du procédé selon l'une quelconque des revendications 1 à 13.

**Patentansprüche**

1. Verfahren zum *In-vitro*-Nachweis/zur *In-vitro*-Vorhersage einer bevorstehenden Geburt, umfassend einen Schritt der gleichzeitigen Suche in einer Probennahme von Vaginal- oder Zervixsekreten nach Markern der Gruppe, bestehend aus intaktem insulinähnlicher-Wachstumsfaktor-Bindungsprotein 1 (intaktem IGFBP-1), gesamtem insulinähnlicher-Wachstumsfaktor-Bindungsprotein 1, umfassend intaktes insulinähnlicher-Wachstumsfaktor-Bindungsprotein 1 und ein N-terminales Fragment des insulinähnlicher-Wachstumsfaktor-Bindungsproteins 1, (Gesamt-IGFBP-1) und Interleukin 6 (IL-6), wobei eine bevorstehende Geburt nachgewiesen/vorhergesagt wird, wenn mindestens zwei der Marker der Gruppe, bestehend aus intaktem insulinähnlicher-Wachstumsfaktor-Bindungsprotein 1 (intaktem IGFBP-1), gesamtem insulinähnlicher-Wachstumsfaktor-Bindungsprotein 1 (Gesamt-IGFBP-1) und Interleukin 6 (IL-6), nachgewiesen werden.

2. Verfahren zum In-vitro-Nachweis/zur In-vitro-Vorhersage einer bevorstehenden Geburt in einer Probe von Vaginal- oder Zervixsekreten, umfassend:

a) Suche nach dem Vorhandensein von intaktem IGFBP-1, Gesamt-IGFBP-1 und IL-6,
b) Zuordnung eines Werts von intaktem IGFBP-1 (IA), wenn intaktes IGFBP-1 in der Probe vorhanden ist, oder von 0, wenn intaktes IGFBP-1 nicht vorhanden ist,
c) Zuordnung eines Werts von IGFBP-1 B ($I_B$), wenn Gesamt-IGFBP-1 in der Probe vorhanden ist, oder von 0, wenn Gesamt-IGFBP-1 nicht vorhanden ist,
wobei der Wert von $I_B$, der zugeordnet wird, wenn Gesamt-IGFBP-1 in der Probe vorhanden ist, kleiner als der oder gleich dem Wert von $I_A$ ist, der zugeordnet wird, wenn intaktes IGFBP-1 in der Probe vorhanden ist,
d) Zuordnung eines Werts von IL-6 ($I_6$), wenn IL-6 in der Probe vorhanden ist, oder von 0, wenn IL-6 nicht vorhanden ist, wobei der Wert $I_6$, der zugeordnet wird, wenn IL-6 in der Probe vorhanden ist, strikt kleiner als die Werte von $I_A$ und $I_B$ ist, die zugeordnet werden, wenn intaktes IGFBP-1 bzw. Gesamt-IGFBP-1 in der Probe vorhanden sind,
e) Berechnen der Bewertungszahl S1 gemäß der folgenden Formel:

$$S1 = I_A + I_B + I_6,$$

wobei ein Wert S1, der größer als der oder gleich dem Wert von $I_A$ bzw. $I_B$ ist, die zugeordnet werden, wenn intaktes IGFBP-1 oder Gesamt-IGFBP-1 in der Probe vorhanden sind, auf eine bevorstehende Geburt hindeutet.

3. Verfahren nach Anspruch 1 oder 2, wobei die Suche mit mindestens einem gegen IL-6 gerichteten Einfangantikörper, mindestens einem gegen intaktes IGFBP-1 gerichteten Einfangantikörper und mindestens einem gegen Gesamt-IGFBP-1 gerichteten Einfangantikörper durchgeführt wird.

4. Verfahren nach Anspruch 2 oder 3, wobei die Suche an einer in einer Pufferlösung verdünnten Probe von Vaginal- oder Zervixsekreten durchgeführt wird.

5. Verfahren nach einem der Ansprüche 1 oder 3, wobei die Suche an einer in einer Pufferlösung verdünnten Probennahme von Vaginal- oder Zervixsekreten durchgeführt wird.

6. Verfahren nach einem der vorhergehenden Ansprüche, wenn sie von Anspruch 1 abhängen, wobei das Verfahren das Einstellen mindestens eines der Parameter, ausgewählt aus dem Volumen, dem pH-Wert, der Ionenstärke des Puffermediums, umfasst.

7. Verfahren nach Anspruch 6, wobei das Verfahren das Einstellen mindestens eines der Parameter, ausgewählt aus der Konzentration der Einfangantikörper, umfasst, wobei das Einstellen spezifisch für IL-6, intaktes IGFBP-1 oder Gesamt-IGFBP-1 ist.

8. Verfahren nach Anspruch 6 oder 7, wobei das Verfahren das Einstellen mindestens eines der Parameter, ausgewählt aus der Konzentration der Einfangantikörper, umfasst, wobei das Einstellen spezifisch für IL-6, intaktes IGFBP-1 oder Gesamt-IGFBP-1 ist.

9. Verfahren nach einem der Ansprüche 6 bis 8, wobei der pH-Wert der Pufferlösung 5 bis 10 beträgt.

10. Verfahren nach einem der vorhergehenden Ansprüche, wobei der Suchschritt einen Sichtbarmachungsschritt umfasst, der mit mindestens einem markierten Anti-intaktes-IGFBP-1-Antikörper, mindestens einem markierten Anti-Gesamt-IGFBP-1-Antikörper und mindestens einem markierten Anti-IL-6-Antikörper durchgeführt wird.

11. Verfahren nach einem der vorhergehenden Ansprüche, wobei intaktes IGFBP-1 ab einer Konzentration von 40 ng/ml nachgewiesen wird.

12. Verfahren nach einem der vorhergehenden Ansprüche, wobei Gesamt-IGFBP-1 ab einer Konzentration von 20 ng/ml intakt und/oder von 10 ng/ml des N-Ter-Fragments von IGFBP-1 nachgewiesen wird.

13. Verfahren nach einem der vorhergehenden Ansprüche, wobei IL-6 ab einer Konzentration von 0,25 ng/ml nachgewiesen wird.

14. Vorrichtung zur Durchführung des Verfahrens nach einem der Ansprüche 1 bis 13, umfassend:

   - eine Probenauftragungszone (1),
   - eine Zone (2), die Anti-intaktes-IGFBP-1-Antikörper, Anti-Gesamt-IGFBP-1-Antikörper und Anti-IL-6-Antikörper, die markiert sind, umfasst
   - eine Sichtbarmachungszone (3), die gegen intaktes IGFBP-1 gerichtete Einfangantikörper umfasst,
   - eine Sichtbarmachungszone (4), die gegen Gesamt-IGFPB-1 gerichtete Einfangantikörper umfasst,
   - eine Sichtbarmachungszone (5), die gegen IL-6 gerichtete Einfangantikörper umfasst.

15. Vorrichtung nach Anspruch 14, wobei die Auftragungszone (1) aus der Gruppe, umfassend ein absorbierendes Papier und einen Glasfaserträger, ausgewählt ist.

16. Vorrichtung nach Anspruch 14 oder 15, wobei die Sichtbarmachungszone (4) und/oder (5) eine Nitrocellulosemembran ist.

17. Vorrichtung nach einem der Ansprüche 14 bis 16, wobei die Zone (2) und/oder (3) ein Glasfaserträger ist.

18. Vorrichtung nach einem der Ansprüche 14 bis 17, wobei die Zonen (1), (2) und/oder (3) ein Glasfaser- oder Nitrocellulosefaserträger sind.

19. Verwendung einer Vorrichtung nach einem der Ansprüche 14 bis 18 zur Durchführung des Verfahrens nach einem der Ansprüche 1 bis 13.

**Claims**

1. Method for the in-vitro detection / prediction of an imminent childbirth, comprising a step of simultaneous search, in a sample of vaginal or cervical secretions, for markers of the group consisting of intact insulin-like growth factor-binding protein 1 (IGFBP-1 intact), total insulin-like growth factor-binding protein 1 comprising the intact insulin-like growth factor-binding protein 1 and a N-Terminal fragment of insulin-like growth factor-binding protein 1 (total IGFBP-1 B) and Interleukin 6 (IL- 6), imminent childbirth is detected / predicted when at least two of the group markers consisting of intact insulin-like growth factor-binding protein 1 (IGFBP-1 intact), total insulin-like growth factor-binding protein 1 (IGFBP-1 total) and interleukin 6 (IL-6), are detected.

2. Method of in vitro detection / prediction of imminent childbirth in a sample of vaginal or cervical secretions comprising:

   a) Searching for the presence of IGFBP-1 intact, IGFBP-1 total and IL-6
   b) Assignment of a IGFBP-1 intact value ($I_A$) if IGFBP-1 intact is present in said sample or 0 if IGFBP-1 A is not present,
   c) Assignment of a IGFBPI total value ($I_B$) if IGFBP-1 total is present in said sample or 0 if IGFBP-1 total is not present, the $I_B$ value assigned if IGFBP-1 total is present in said sample being lower or equal to the value of $I_A$ assigned when IGFBP-1 intact is present in said sample
   d) Assignment of a IL-6 value ($I_6$) if IL-6 is present in said sample or of 0 if IL-6 is not present, the $I_6$ value assigned when IL-6 is present in said sample being strictly lower than the values of $I_A$ and $I_B$ assigned when IGFBP-1 intact and IGFBP-1 total are respectively present in said sample,
   e) Calculation of S1 score according to the following formula:

$$S_1 = I_A + I_B + I_6$$

   a value of S 1 greater than or equal to the value of $I_A$ or $I_B$, respectively assigned when IGFBP-1 intact or IGFBP-1 total is present in said sample, indicating an imminent delivery.

3. Method according to claim 1 or 2, wherein the research is carried out with at least one capture antibody directed against IL-6, at least one capture antibody directed against IGFBP-1 intact and at least one capture antibody directed against IGFBP-1 total.

4. Method according to claim 2 or 3, wherein the research is carried out using a sample of vaginal or cervical secretions diluted in a buffer solution.

5. Method according to any one of claims 1 or 3, wherein the research is carried out using a sample of vaginal or cervical secretions diluted in a buffer solution.

6. Method according to any one of the preceding claims when they are dependent on claim 1 wherein the method comprises adjusting at least one of the parameters selected from the volume, the pH, the ionic strength of the buffer medium.

7. Method according to claim 6 wherein the method comprises the adjustment at least one of the parameters selected from the concentration of capture antibodies, said adjustment being specific for IL-6, IGFBP-1 intact or IGFBP-1 total.

8. Method according to claim 6 or 7, wherein the method comprises adjusting at least one of the parameters selected from the concentration of capture antibodies, said adjustment being specific for IL-6, IGFBP-1 intact and/or IGFBP-1 total.

9. Method according to any one of claims 6 to 8 wherein the pH of the buffer solution is from 5 to 10.

10. Method according to any one of the preceding claims, wherein said research step comprises a revealing step carried out with at least one labeled anti-IGFBP-1 intact antibody, at least one labeled anti-IGFBP-1 total antibody and at least one labeled anti-IL-6 antibody.

11. Method according to any preceding claim, wherein IGFBP-1 intact is detected starting at a concentration of 40 ng / ml.

**12.** Method according to any preceding claim, wherein IGFBP-1 total is detected from a concentration of 20 ng / mL intact and / or 10 ng / mL of the N-Ter fragment of IGFBP-1.

**13.** Method according to any preceding claim, wherein IL-6 is detected starting at a concentration of 0.25 ng / ml.

**14.** Device for implementing the method according to any one of claims 1 to 13 comprising:

- a sample-depositing zone (1),
- a zone (2) comprising labeled anti-IGFBP 1 intact antibodies, labeled anti-IGFBP 1 total antibodies and labeled anti-IL-6 antibodies,
- a revealing zone (3) comprising capture antibodies directed against IGFBP 1 intact,
- a revealing zone (4) comprising capture antibodies directed against IGFPB-1 total,
- a revealing zone (5) comprising capture antibodies directed against IL-6.

**15.** Device according to claim 14, wherein the deposition zone (1) is selected from the group comprising an absorbent paper and a glass fiber support.

**16.** Device according to claim 14 or 15, wherein the revealing zone (4) and / or (5) is a nitrocellulose membrane.

**17.** Device according to any one of claims 14 to 16, wherein zone (2) and / or (3) is a fiberglass support.

**18.** Device according to any one of claims 14 to 17, wherein zones (1), (2) and / or (3) is a fiberglass or nitrocellulose support.

**19.** Use of the device according to any one of claims 14 to 18, for implementing the method according to any one of claims 1 to 13.

A

(1)

(2)

(3)

(4)

(5)

(6)

(7)

(8)

IL-6

C

T

S

7.0 mm

34.5 mm

B

Figure 1

A

B

Figure 2

A

(12)

(9)

(13)

(14)

(10)

(16)

(11)

IGFBP1 B

7.0 mm

C

T

S

34.5 mm

B

Figure 3

Écouvillon avec du Nylon CE 0123 stérile.

# Figure 4

**Évalués pour l'admissibilité**
(n=117)

**Exclue** (n=19)
- Ne souhaitait pas participer (n=3)
- Ne remplissait pas les critères d'inclusion (n=16)
  - RPM (n=7)
  - Coït dans les 24 heures suivant l'évaluation (n=5)
  - cerclage cervical (n=2)
  - autre (n=2)

**Inscrits** (n=98)

**Paramètres évalués**

1.) Nombre d'accouchements <37 SA
2.) Nombre d'accouchements dans les 14 jours à compter de l'admission

**Analyse** (n=95)
- Nombre d'accouchements <37 SA (n=25)
  - Nombre d'accouchements ≤14 jours de l'admission (n=23)
  - Nombre d'accouchements >14 jours de l'admission (n=2)
- Nombre d'accouchements ≥37 SA (n=70)
  - Nombre d'accouchements ≤ 14 jours de l'admission (n=10)
  - Nombre d'accouchement s>14 jours de l'admission (n=60)
- Exclus de l'analyse (n=3)
  - Résultats non valides (n=2)
  - Accouchement iatrogénique avant 14 jours de l'admission (n=1)

## Figure 5

Figure 6

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1651626

**Littérature non-brevet citée dans la description**

- **RUTANEN et al.** *Biochem Biophys Res Commun,* 1988, vol. 152, 208
- **PEKONEN et al.** *J immunoassay,* 1989, vol. 10, 325-337
- **BEER et al.** Qualification of cellulose nitrate membranes for lateral-flow assays. *IVD technology,* Janvier 2002
- **ANNE HARWOOD PERUSKI et al.** Immunological Methods for Détection and Identification of Infectious Disease and Biological Warfare Agents. *Clinical and diagnostic laboratory immunology,* Juillet 2003, 506-513
- **EROGLU D ; YANIK F ; OKTEM M ; ZEYNELOGLU HB ; KUSCU E.** *Prediction of preterm delivery among women with threatened preterm labor,* 2007, vol. 64 (2), 109-16
- **BERGHELLA V.** Novel developments on cervical length screening and progesterone for preventing preterm birth. *BJOG.,* Janvier 2009, vol. 116 (2), 182-7
- **BERGHELLA V ; BAXTER JK ; HENDRIX NW.** Cervical assessment by ultrasound for preventing preterm delivery. *Cochrane Database Syst Rev.,* 31 Janvier 2013, vol. 1, CD007235
- **GOEPFERT AR ; GOLDENBERG RL ; ANDREWS WW ; HAUTH JC ; MERCER B et al.** The Preterm Prédiction Study: association between cervical interleukin 6 concentration and spontaneous preterm birth. National Institute of Child Health and Human Development Maternal-Fetal Medicine Units Network. *Am J Obstet Gynecol.,* Février 2001, vol. 184 (3), 483-8

- **LEITICH H ; EGARTER C ; KAIDER A ; HOHLAG-SCHWANDTNER M ; BERGHAMMER P ; HUSSLEIN P.** Cervicovaginal fetal fibronectin as a marker for preterm delivery: a meta-analysis. *Am J Obstet Gynecol.,* Mai 1999, vol. 180 (5), 1169-76
- **GOLDENBERG RL ; MERCER BM ; MEIS PJ ; COPPER RL ; DAS A ; MCNELLIS D.** The preterm prédiction study: fetal fibronectin testing and spontaneous preterm birth. NICHD Maternai Fetal Medicine Units Network. *Obstet Gynecol.,* Mai 1996, vol. 87 (5), 643-8
- **MCLAREN JS ; HEZELGRAVE NL ; AYUBI H ; SEED PT ; SHENNAN AH.** Prediction of spontaneous preterm birth using quantitative fetal fibronectin after recent sexual intercourse. *Am J Obstet Gynecol.,* Janvier 2015, vol. 212 (1), 89.e1-5
- **KURKINEN-RÄTY M ; RUOKONEN A ; VUOPALA S ; KOSKELA M ; RUTANEN EM ; KÄRKKÄINEN T ; JOUPPILA P.** Combination of cervical interleukin-6 and -8, phosphorylated insulin-like growth factor-binding protein-1 and transvaginal cervical ultrasonography in assessment of the risk of preterm birth. *BJOG,* Août 2001, vol. 108 (8), 875-81
- **HONEST H ; FORBES CA ; DUREE KH et al.** Screening to prevent spontaneous preterm birth: systematic reviews of accuracy and effectiveness literature with economic modelling. *Health Technol Assess,* 2009, vol. 13, 1-627
- **RIBONI et al.** Combination of biochemical markers in predicting pre-term delivery. *Archives of Gynecology and Obstetrics,* 2011, vol. 285 (1), 61-66